# EUROPEAN PATENT SPECIFICATION

(11) **EP 3 997 086 B1**
(45) Date of publication and mention of the grant of the patent: **05.06.2024**
(21) Application number: 20740271.0
(22) Date of filing: 09.07.2020
(51) Int. Cl.: C07D 413/12, C07D 217/06, C07D 217/14, C07D 217/16, A61P 35/00, A61K 31/472, A61K 31/4725

(54) **TETRAHYDROISOQUINOLINE COMPOUNDS**
TETRAHYDROISOCHINOLINVERBINDUNGEN
COMPOSÉS DE TÉTRAHYDROISOQUINOLINE

(30) Priority: 09.07.2019 EP 19382584
(43) Date of publication of application: 18.05.2022
(73) Proprietor: Allinky Biopharma, 28049 Madrid (ES)
(72) Inventor: VEGA GARCÍA, Miguel, 28049 Madrid (ES); CAMPOS MUELAS, Pedro, 28049 Madrid (ES); CARRASCO ROMERO, Esther, 28049 Madrid (ES); BURGUETE PÉREZ, Asunción, 28049 Madrid (ES); GÓMEZ GUTIÉRREZ, Patricia, 28049 Madrid (ES); PÉREZ GONZÁLEZ, Juan Jesús, 28049 Madrid (ES); MESSEGUER PEYPOCH, Ángel, 28049 Madrid (ES); ALARCÓN SÁNCHEZ, Balbino José, 28049 Madrid (ES)
(74) Representative: Hoffmann Eitle
(86) International application number: PCT/EP2020/069403
(87) International publication number: WO 2021/005165

(56) References cited:
- WO-A1-2011/005636
- WO-A1-2016/033105
- WO-A1-2019/137985
- US-A- 3 905 982
- NAGORE I. MARÍN-RAMOS ET AL: "Blocking Ras inhibition as an antitumor strategy", SEMINARS IN CANCER BIOLOGY., 1 February 2018 (2018-02-01), XP055479942, US ISSN: 1044-579X, DOI: 10.1016/j.semcancer.2018.01.017
- YUANXIANG WANG ET AL: "Targeting Mutant KRAS for Anticancer Therapeutics: A Review of Novel Small Molecule Modulators", JOURNAL OF MEDICINAL CHEMISTRY, vol. 56, no. 13, 23 April 2013 (2013-04-23), pages 5219-5230, XP055458688, US ISSN: 0022-2623, DOI: 10.1021/jm3017706
- CAPILLA A SERGI ET AL: "Substituted tetrahydroisoquinolines: synthesis, characterization, antitumor activity and other biological properties", EUROPEAN JOURNAL OF MEDICINAL CHEMISTRY, ELSEVIER, AMSTERDAM, NL, vol. 145, 3 January 2018 (2018-01-03), pages 51-63, XP085414801, ISSN: 0223-5234, DOI: 10.1016/J.EJMECH.2017.12.098

## Description

### Field of the invention.

The present invention relates to a novel class of tetrahydroisoquinoline compounds and to compositions comprising the same. The compounds and pharmaceutical compositions of the present invention can be used as medicaments in the treatment of cancer.

### Background of the invention

Cancer genome sequencing efforts over the past 10 to 15 years have led to the identification of numerous oncogenes responsible for the development and maintenance of human cancer. Despite the identification of more than 500 validated cancer genes the three RAS genes HRAS, NRAS and KRAS still constitute the most frequently mutated oncogene family in human cancer.

When RAS is 'switched on' by incoming signals, it subsequently switches on other proteins, which ultimately turn on genes involved in cell growth, differentiation and survival. Mutations in *ras* genes can lead to the production of permanently activated RAS proteins. As a result, this can cause unintended and overactive signaling inside the cell, even in the absence of incoming signals.

Because these signals result in cell growth and division, overactive RAS signaling can ultimately lead to cancer. The 3 RAS genes (HRas, KRas, and NRas) are the most common oncogenes in human cancer; mutations that permanently activate RAS are found in 20% to 25% of all human tumors and up to 90% in certain types of cancer.

Cancers harboring RAS mutations remained essentially untreatable more than 30 years after the initial discovery of the oncogene. Thus, for many years RAS was considered to be "undruggable".

Among HRAS, NRAS and KRAS, KRAS is the most frequently mutated RAS isoform having been shown to be mutated in 90% of pancreatic adenocarcinoma, 45% of colon rectal cancers and 35% of lung adenocarcinoma. KRAS mutations have been associated with increased tumorigenicity and poor prognosis.

To date, different types of drugs are used as anticancer drugs and cisplatin represents one of the most popular. Cisplatin is used to treat various types of cancers, including sarcomas, some carcinomas (e.g., small cell lung cancer, squamous cell carcinoma of the head and neck and ovarian cancer), lymphomas, bladder cancer, cervical cancer and germ cell tumors. Even though it resulted to be very effective in some kinds of cancer (such as testicular cancer) it shows a number of side-effects that can limit its use. Furthermore, according to the mechanism of action proposed for cisplatin, it should interfere with DNA replication, killing the fastest proliferating cells, which in theory are carcinogenic. However, cisplatin is not really selective towards carcinogenic cells.

International application number WO2019/137985 provides some compounds acting as anti-cancer drugs having low toxicity.

However, there is still a need to provide further novel compounds acting as anti-cancer drugs and, at the same time, having low toxicity.

### Summary of the invention

The present invention provides a novel class of compounds having Formula I and/or Formula II, which includes enantiomers and pharmaceutically acceptable salts thereof. The compounds of the present invention selectively and effectively inhibit RAS proteins, and particularly KRAS proteins, thereby representing excellent anti-cancer drugs useful in the treatment of a variety of cancers, such as large intestine cancer, colon cancer, rectal cancer, pancreatic cancer, breast cancer, multiple myeloma, leukemia and lung cancer. Compared to known compounds used in the treatment of cancer, the compounds of the present invention also exhibit lower toxicity.

The compounds of the present invention are compounds of Formula I, enantiomers or pharmaceutically acceptable salts thereof: 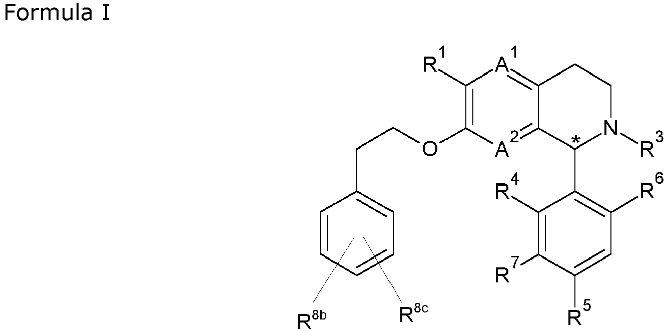 wherein
A¹ is selected from CR² and N;
A² is selected from CH and N;
R¹ is OR^{x},
R^{x} is C₁₋₄ alkyl,
   or H;
R² is H, C₁₋₄ alkyl, C₁₋₄ haloalkyl, halogen, OC₁₋₄ alkyl, OC₁₋₄ haloalkyl, OH or NH₂;
R³ is -(CH₂)n³-C(Y³)-(X³)m³-(CH₂)ₖ³-R^{3a},
n³ is an integer in the range of 0 to 2,
Y³ is O,
X³ is NH, or O,
m³ is 0 or 1,
k³ is 0 or 1,
R^{3a} is C₁₋₄ alkyl, C₁₋₄ haloalkyl, or HET³,
Het³ is oxazolyl, isoxazolyl, thiazolyl, isothiazolyl, or pyridinyl,
R⁴ is halogen, C₁₋₄ alkyl, C₁₋₄ haloalkyl, OC₁₋₄ alkyl, or OC₁₋₄ haloalkyl,
R⁵ is H, halogen, OC₁₋₄ alkyl, OH, C₁₋₄ alkyl, C₁₋₄ haloalkyl, or OC₁₋₄ haloalkyl,
R⁶ is H, OH, or halogen;
R⁷ is H, halogen, OH, or C₁₋₄ alkyl;
R^{8b} and R^{8c} are independently selected from the group consisting of OH, C₁₋₄ alkyl, OC₁₋₄ alkyl, halogen, NO₂, and NH₂.

### Detailed description of the invention

### Definitions

In the present context, the term "C₁₋₄ alkyl" is intended to mean a linear or branched hydrocarbon group having 1 to 4 carbon atoms, such as methyl, ethyl,n-propyl,isopropyl,n-butyl,iso-butyl, sec-butyl,and tert-butyl.

Similarly, the term "C₂₋₄ alkenyl" is intended to cover linear or branched hydrocarbon groups having 2 to 4 carbon atoms and comprising a double bond. Examples of alkenyl groups are vinyl, allyl, and butenyl. Preferred examples of alkenyl are vinyl and allyl, especially allyl.

In the present context the term "C₂₋₄ alkynyl" is intended to mean a linear or branched hydrocarbon group having 2 to 4 carbon atoms and containing a triple bond. Illustrative examples of C₂₋₄ alkynyl groups include acetylene, propynyl, butynyl, as well as branched forms of these. The position of unsaturation (the triple bond) may be at any position along the carbon chain. More than one bond may be unsaturated such that the "C₂₋₄ alkynyl" is a di-yne as is known to the person skilled in the art.

In the present context, the term "C₁₋₄ alkanediyl" is intended to mean a divalent linear or branched hydrocarbon group having 1 to 4 carbon atoms, such as methanediyl, ethanediyl,propanediyl, or butanediyl.

Similarly, the term "C₂₋₄ alkenediyl" is intended to cover divalent linear or branched hydrocarbon groups having 2 to 4 carbon atoms and comprising a double bond.

In the present context the term "C₂₋₄ alkynediyl" is intended to mean a divalent linear or branched hydrocarbon group having 2 to 4 carbon atoms and containing a triple bond.

Herein, the term "halogen" includes fluoro, chloro, bromo, and iodo, more particularly, fluoro, chloro and bromo.

In the text, the term "C₁₋₄ haloalkyl" is intended to mean a linear or branched hydrocarbon group having 1 to 4 carbon atoms that is substituted with one or more halogen atoms, such as trifluoromethyl or fluoromethyl.

Similarly, the term "C₂₋₄ haloalkenyl" is intended to cover linear or branched hydrocarbon groups having 2 to 4 carbon atoms and comprising a double bond and substituted with one or more halogen atoms. Examples of haloalkenyl groups are 1,1-dichloroprop-1-en-2-yl and (Z)-3-fluoroprop-1-en-1-yl.

In the text the term "C₂₋₄ haloalkynyl" is intended to mean a linear or branched hydrocarbon group having 2 to 4 carbon atoms and containing a triple bond and substituted with one or more halogen atoms, such as 3-chloroprop-1-yn-1-yl.

In the present context the term "aromatic ring or ring system" is intended to mean a fully or partially aromatic carbocyclic ring or ring system, such as phenyl, naphthyl, 1,2,3,4-tetrahydronaphthyl, anthracyl, phenanthracyl, pyrenyl, benzopyrenyl, fluorenyl and xanthenyl.

The term "heteroaromatic ring or ring system" is intended to mean a fully or partially aromatic carbocyclic ring or ring system where one or more of the carbon atoms have been replaced with heteroatoms, e.g. nitrogen (=N- or -NH-), sulphur, and/or oxygen atoms. Examples of such heteroaromatic ring or ring system groups are oxazolyl, isoxazolyl, thiazolyl, isothiazolyl, pyrrolyl, imidazolyl, pyrazolyl, pyridinyl, pyrimidinyl, pyrazinyl, pyridazinyl, triazinyl, coumaryl, furyl, thienyl, quinolyl, benzothiazolyl, benzotriazolyl, benzodiazolyl, benzooxozolyl, phthalazinyl, phthalanyl, triazolyl, tetrazolyl, isoquinolyl, acridinyl, carbazolyl, dibenzazepinyl, indolyl, benzopyrazolyl and phenoxazonyl.

In the present context, the term "heterocyclic ring or ring system" is intended to mean a non-aromatic carbocyclic ring or ring system where one or more of the carbon atoms have been replaced with heteroatoms, e.g. nitrogen (=N- or -NH-), sulphur, and/or oxygen atoms. Examples of such heterocyclic groups are imidazolidine, piperazine, hexahydropyridazine, hexahydropyrimidine, diazepane, diazocane, pyrrolidine, piperidine, azepane, azocane, aziridine, azirine, azetidine, pyroline, tropane, oxazinane (morpholine), azepine, dihydroazepine, tetrahydroazepine, hexahydroazepine, oxazolane, oxazepane, oxazocane, thiazolane, thiazinane, thiazepane, thiazocane, oxazetane, diazetane, thiazetane, tetrahydrofuran, tetrahydropyran, oxepane, tetrahydrothiophene, tetrahydrothiopyrane, thiepane, dithiane, dithiepane, dioxane, dioxepane, oxathiane and oxathiepane.

In the present context, the term "optionally substituted" is intended to mean that the group in question may be substituted at least once. Furthermore, the term "optionally substituted" may also mean that the group in question is unsubstituted.

The compounds of the present invention can be in a free form or in the form of a pharmaceutically acceptable salt. In the context of the present invention, the term "pharmaceutically acceptable salt" is to be understood as a salt formed with either a base or an acid, wherein the resulting counter-ion does not significantly add to the toxicity of the compound of the present invention.

Examples of pharmaceutically acceptable salts include inorganic acid salts such as hydrochloride, sulfate, nitrate, phosphate or hydrobromide, etc., organic acid salts such as acetate, fumarate, oxalate, citrate, methanesulfonate, benzenesulfonate, p-toluenesulfonate or maleate, etc. Also, when the compound has a substituent such as carboxyl group, there may be mentioned a salt with a base (for example, alkali metal salt such as sodium salt, potassium salt, etc. or alkaline earth metal salt such as calcium salt, etc.).

### Compounds

The compounds of the invention are compounds of Formula I, enantiomers or pharmaceutically acceptable salts thereof: 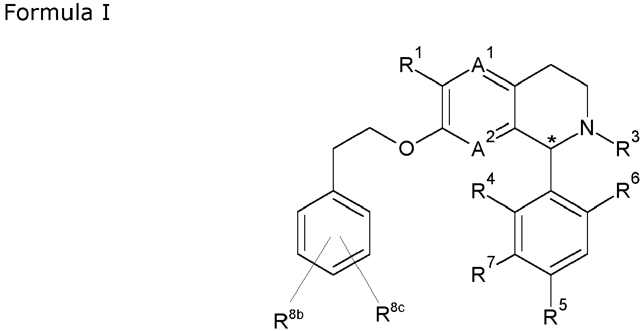 wherein
A¹ is selected from CR² and N;
A² is selected from CH and N;
R¹ is OR^{x}, such as OCH₃,
R^{x} is C₁₋₄ alkyl,
or H, such as CH₃, or H,
R² is H, C₁₋₄ alkyl, C₁₋₄ haloalkyl, halogen, OC₁₋₄ alkyl, OC₁₋₄ haloalkyl, OH or NH₂, such as H, CH₃, or OCH₃, particularly H or OCH₃, more particularly H; R³ is -(CH₂)n³-C(Y³)-(X³)m³-(CH₂)ₖ³-R^{3a},
n³ is an integer in the range of 0 to 2, such as 0 or 2,
Y³ is O,
X³ is NH or O, particularly NH,
m³ is 0 or 1,
k³ is 0 or 1, R^{3a} is C₁₋₄ alkyl, C₁₋₄ haloalkyl, or Het³, in particular CF₃,
Het³ is oxazolyl, isoxazolyl, thiazolyl, isothiazolyl, or pyridinyl, particularly oxazolyl, thiazolyl, or pyridinyl, more particularly oxazol-4-yl, thiazol-4-yl, or pyridin-4-yl,
R⁴ is halogen, C₁₋₄ alkyl, C₁₋₄ haloalkyl, OC₁₋₄ alkyl, or OC₁₋₄ haloalkyl, such as halogen or C₁₋₂ alkyl, particularly Cl, F, or C₁₋₂ alkyl, more particularly, Cl, F, or CH₃, even more particularly Cl or CH₃, such as CH₃;
R⁵ is H, halogen, OC₁₋₄ alkyl, OH, C₁₋₄ alkyl, C₁₋₄ haloalkyl, or OC₁₋₄ haloalkyl, particularly halogen, C₁₋₂ haloalkyl, C₁₋₂ alkyl, or OC₁₋₂ alkyl, more particularly CH₃, CF₃, OCH₃, F or Cl, even more particularly CH₃ or CF₃, such as CH₃;
R⁶ is H, OH, or halogen, such as H or OH, more particularly H;
R⁷ is H, halogen, OH, C₁₋₄ alkyl, such CH₃, particularly H;
R^{8b} and R^{8c} are independently selected from the group consisting of OH, C₁₋₄ alkyl, OC₁₋₄ alkyl, halogen, NO₂, or particularly, OH, OCH₃, and F, even more particularly OH and F, such as OH;

In one embodiment, R^{8b} and R^{8c} are independently selected from the group consisting of OH, OCH₃, NH₂, NO₂, and halogen. In a further embodiment, R^{8b} and R^{8c} are independently selected from the group consisting of OH, OCH₃, NH₂, NO₂, and F. In yet a further embodiment, R^{8b} and R^{8c} are independently selected from the group consisting of OCH₃ and NH₂. In a further embodiment, at least one of R^{8b} and R^{8c} is in the meta position relative to the ethyl-oxy group to which the phenyl group is bound. In still a further embodiment, the phenyl ring is a phen-1-yl substituted in positions 3 and 4.

R³ is -(CH₂)ₙ³-C(Y³)-(X³)ₘ³-(CH₂)ₖ³-R^{3a}. In one embodiment, X³ is NH. In a further variation of this embodiment, n³ is 0. In another variation of these embodiments, m³ is 1. In still another variation of these embodiments, n³ is 0 and m³ is 1. In yet another variation of these embodiments, R^{3a} is oxazolyl, methyl, or trifluoromethyl.

In a different variation of the embodiment, n³ is 2 and m³ is 0,

In still a further variation of these embodiments having different variants of R³, k³ is 1. In one embodiment, A¹ is CR². In a further embodiment, A¹ is CH. In another embodiment, A² is CH. In yet another embodiment, A¹ is CH and A² is CH.

R¹ is OR^{x}. In one embodiment, R¹ is OCH₃.

R^{x} is C₁₋₄ alkyl, or H. In one embodiment, R^{x} is CH₃ or H.

R² is H, C₁₋₄ alkyl, C₁₋₄ haloalkyl, halogen, OC₁₋₄ alkyl, OC₁₋₄ haloalkyl, OH or NH₂.

In one embodiment, R² is H or O-C₁₋₄ alkyl. In another embodiment, R² is H.

R⁴ is halogen, C₁₋₄ alkyl, C₁₋₄ haloalkyl, OC₁₋₄ alkyl, or OC₁₋₄ haloalkyl.

In one embodiment, R⁴ is halogen or C₁₋₂ alkyl. In a further embodiment, R⁴ is Cl, F, or C₁₋₂ alkyl. In still a further embodiment, R⁴ is Cl, F, or CH₃. In another embodiment, R⁴ is Cl or CH₃. In yet another embodiment, R⁴ is CH₃.

R⁵ is H, halogen, OC₁₋₄ alkyl, OH, C₁₋₄ alkyl, C₁₋₄ haloalkyl, or OC₁₋₄ haloalkyl. In one embodiment, R⁵ is halogen, C₁₋₂ alkyl, OC₁₋₂ alkyl, or C₁₋₂ haloalkyl. In a further embodiment, R⁵ is halogen, CF₃, CH₃, or OCH₃. In still a further embodiment, R⁵ is F, Cl, CF₃, CH₃, or OCH₃. In yet a further embodiment, R⁵ is CH₃.

R⁶ is H, OH, or halogen.

In one embodiment, R⁶ is H or OH. In a further embodiment,

R⁶ is H.

R⁷ is H, halogen, OH, or C₁₋₄ alkyl.

In one embodiment, R⁷ is H, or CH₃.

In a further embodiment, R⁷ is H.

In a further embodiment according to the invention:
A¹ is selected from CR² and N, preferably CR²;
A² is selected from CH and N, preferably CH;
R¹ is OCH₃;
R² is H, C₁₋₄ alkyl, C₁₋₄ haloalkyl, halogen, OC₁₋₄ alkyl, OC₁₋₄ haloalkyl, OH or NH₂, such as H, CH₃, or OCH₃, particularly H or OCH₃, most preferably R² is H;
R³ is -(CH₂)n³-C(Y³)-(X³)m³-(CH₂)ₖ³-R^{3a},
n³ is 0,
Y³ is O,
X³ is NH,
m³ is 1,
k³ is 0 or 1,
R^{3a} is oxazolyl, isoxazolyl, CH₃, CF₃ or CH(CH₃)CF₃ (including (*R*)-CH(CH₃)CF₃, (*S*)-CH(CH₃)CF₃ or mixture thereof);
R⁴ is halogen, C₁₋₄ alkyl, C₁₋₄ haloalkyl,
OC₁₋₄ alkyl, or OC₁₋₄ haloalkyl, such as halogen or C₁₋₂ alkyl, particularly Cl, F, or C₁₋₂ alkyl, more particularly, Cl, F, or CH₃, even more particularly Cl or CH₃, such as CH₃;
R⁵ is H, halogen, OC₁₋₄ alkyl, OH, C₁₋₄ alkyl, C₁₋₄ haloalkyl, or OC₁₋₄ haloalkyl, particularly halogen, C₁₋₂ haloalkyl, C₁₋₂ alkyl, or OC₁₋₂ alkyl, more particularly CH₃, CF₃, OCH₃, F or Cl, even more particularly CH₃ or CF₃, such as CH₃;
R⁶ is H, OH, or halogen, such as H or OH, more particularly H;
R⁷ is H, halogen, OH, or C₁₋₄ alkyl, such as H, CH₃, particularly H;
R^{8b} and R^{8c} are independently selected from the group consisting of OH, OCH₃, NH₂, NO₂, Cl and F.

In a preferred embodiment according to the invention:
A¹ is selected from CR² and N, preferably CR²;
A² is selected from CH and N, preferably CH;
R¹ is OCH₃;
R² is H, C₁₋₄ alkyl, C₁₋₄ haloalkyl, halogen, OC₁₋₄ alkyl, OC₁₋₄ haloalkyl, OH or NH₂, such as H, CH₃, or OCH₃, particularly H or OCH₃, most preferably R² is H;
R³ is -(CH₂)ₙ³-C(Y³)-(X³)ₘ³-(CH₂)ₖ³-R^{3a},
n³ is 0,
Y³ is O,
X³ is NH,
m³ is 1,
k³ is 0 or 1,
R^{3a} is oxazolyl, isoxazolyl, CH₃, CF₃ or CH(CH₃)CF₃ (including (*R*)-CH(CH₃)CF₃, (*S*)-CH(CH₃)CF₃ or mixture thereof);
R⁴ is CH₃;
R⁵ is CH₃, CF₃, OCH₃, F, Cl, or Br;
R⁶ is H;
R⁷ is H; and
R^{8b} and R^{8c} are independently selected from the group consisting of OH, OCH₃, NH₂, NO₂, Cl and F.

In a particular embodiment of the invention, the compounds of the invention are compounds of Formula II, enantiomers or pharmaceutically acceptable salts thereof: 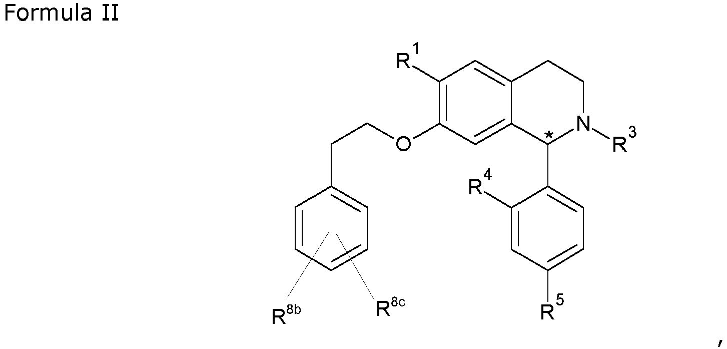 wherein R¹, R³, R⁴, R⁵, R^{8b}, and R^{8c} are as defined above for Formula I. In a further embodiment, at least one of R^{8b} and R^{8c} is in the meta position relative to the ethyl-oxy group to which the phenyl group is bound. In still a further embodiment, the phenyl ring is a phen-1-yl substituted in positions 3 and 4.

In a preferred embodiment, the compound of the invention is selected from the group consisting of compounds 1-52, enantiomers, and pharmaceutically acceptable salts thereof:

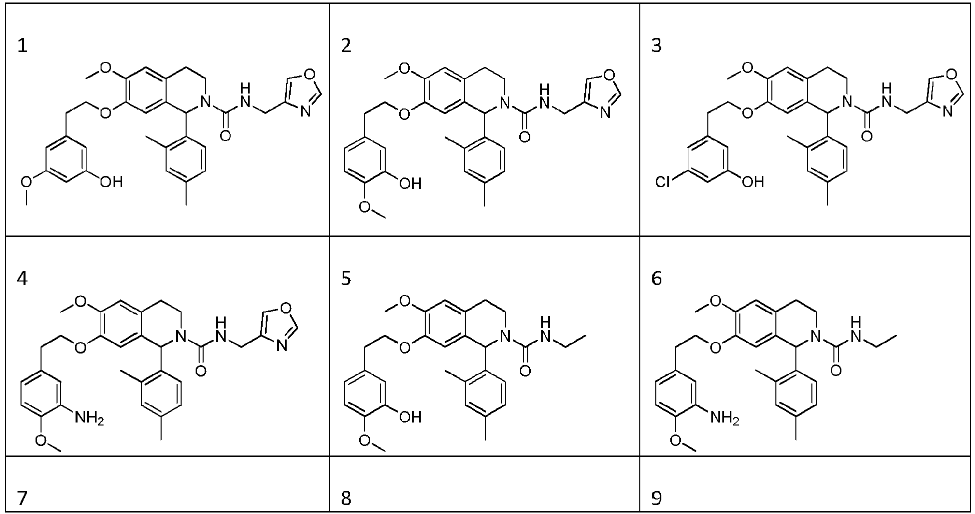

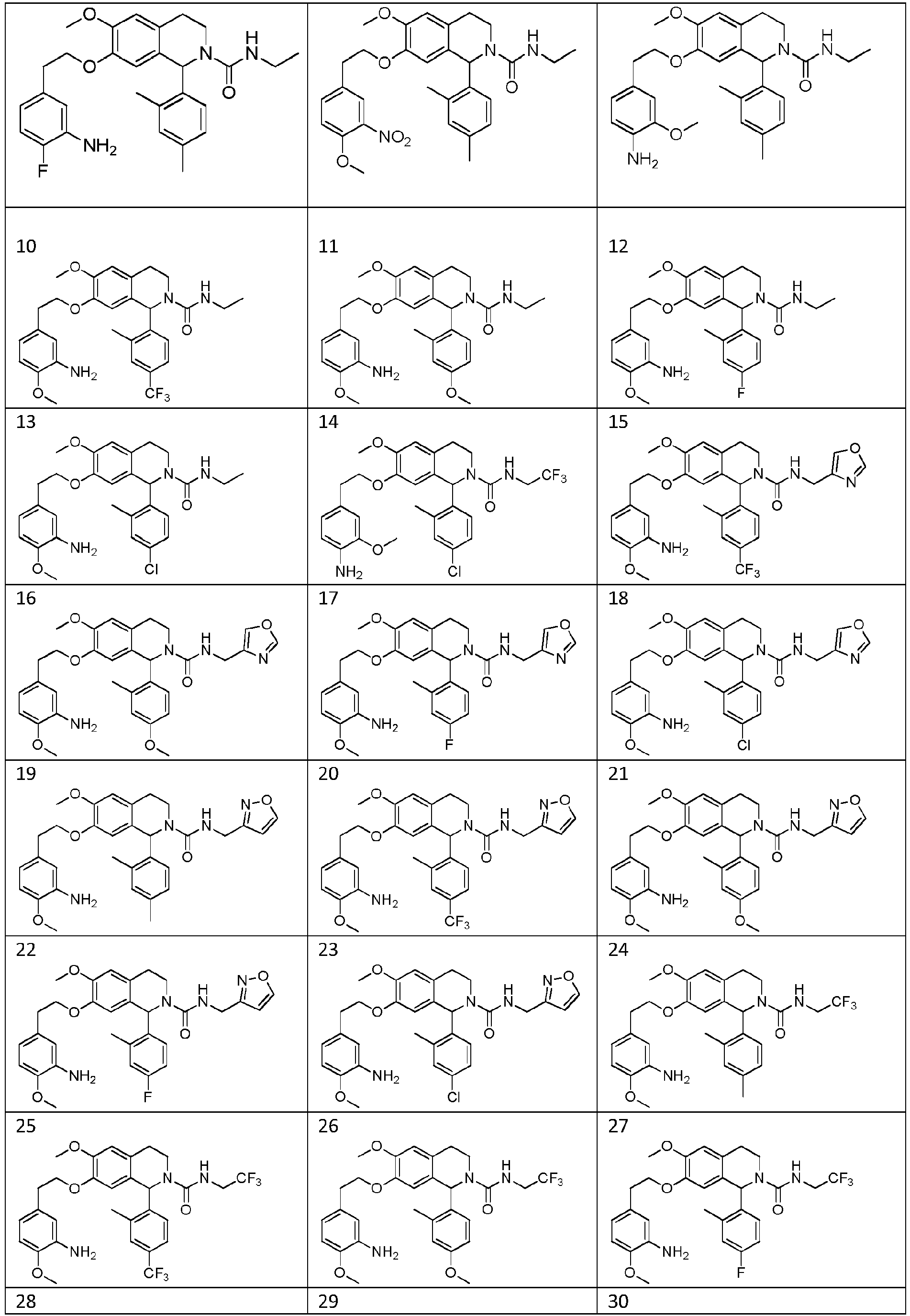

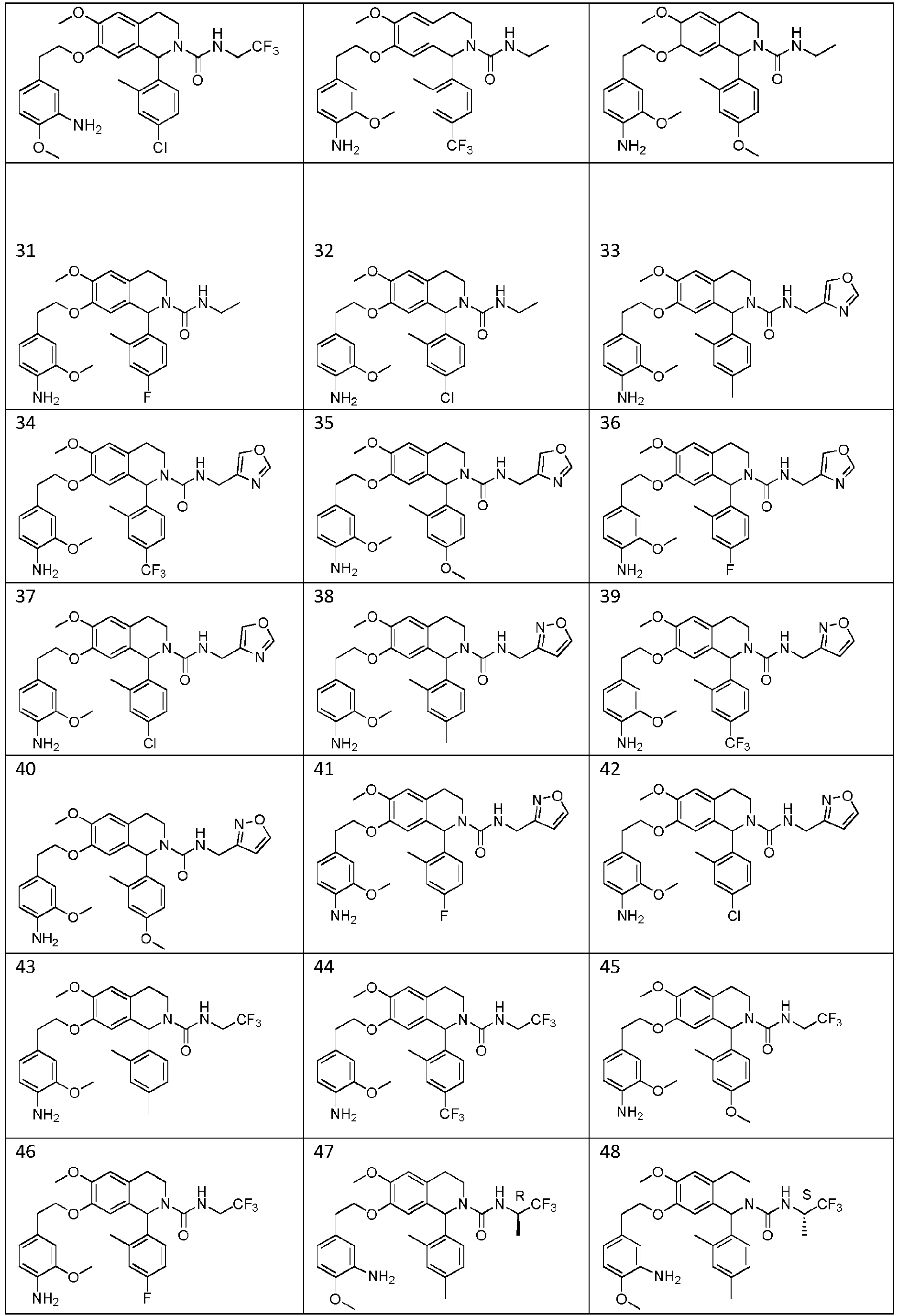

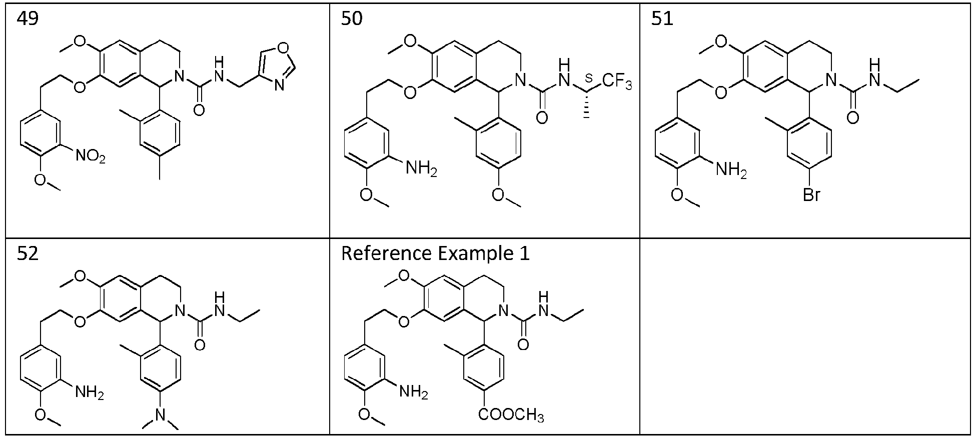

### Pharmaceutical formulation

The compounds of the present invention are intended for use as a medicament. The compounds of the invention may in principle be applied on their own, but they are preferably formulated with a pharmaceutically acceptable carrier. A pharmaceutically acceptable carrier is an inert carrier suitable for each administration method, and can be formulated into conventional pharmaceutical preparation (tablets, granules, capsules, powder, solution, suspension, emulsion, injection, infusion, etc.). As such a carrier there may be mentioned, for example, a binder, an excipient, a lubricant, a disintegrant and the like, which are pharmaceutically acceptable. When they are used as an injection solution or an infusion solution, they can be formulated by using distilled water for injection, physiological saline, an aqueous glucose solution.

The administration method of the compounds of the present invention is not particularly limited, and a usual oral or parenteral administration method (intravenous, intramuscular, subcutaneous, percutaneous, intranasal, transmucosal, enteral, etc.) can be applied.

The dosage of the tetrahydroisoquinoline derivatives or a pharmaceutically acceptable salts thereof of the present invention may optionally be set in a range of an effective amount sufficient for showing a pharmacological effect, in accordance with the potency or characteristics of the compound to be used as an effective ingredient. The dosage may vary depending on administration method, age, body weight or conditions of a patient.

### Pharmaceutical utility

The compounds of the invention are intended for the treatment of cancer. Hence, in one aspect, the invention concerns a compound or composition according to the invention for use in the treatment of cancer. In particular Ras-driven cancer, Ras genes being the first oncogenes identified in human cancer cells. In one embodiment, the invention concerns a compound or composition according to the invention for use in the treatment of leukemias, lymphomas, myelomas, colorectal cancer, pancreatic cancer, breast cancer and lung cancer, among other types of cancer.

### Preparation of compounds

The substituted tetrahydroisoquinolines **H** of the present invention are generally prepared in five steps as outlined in Scheme 1.

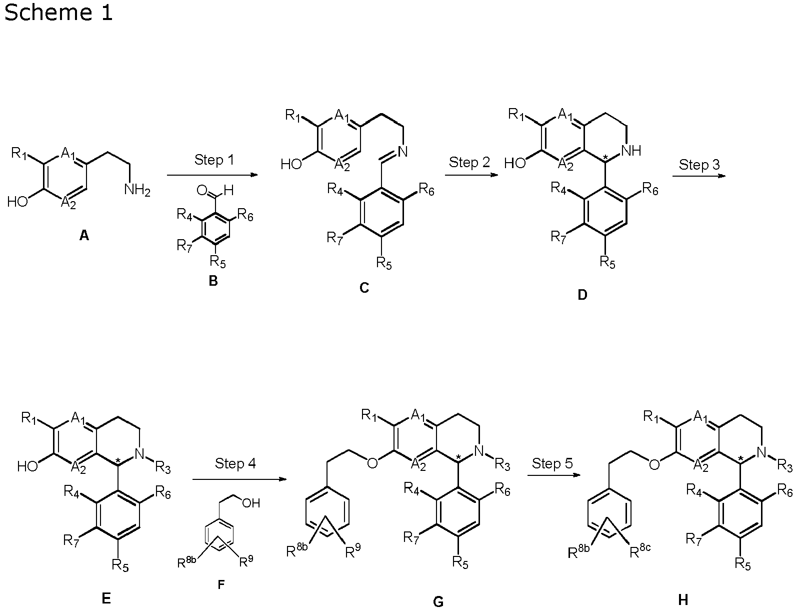

Some of the compounds according to the present invention do not require additional transformations after the corresponding alcohol is added to the molecule (step 4) and those substituted tetrahydroisoquinolines **G** of the present invention are generally prepared in four steps as outlined in Scheme 1b.

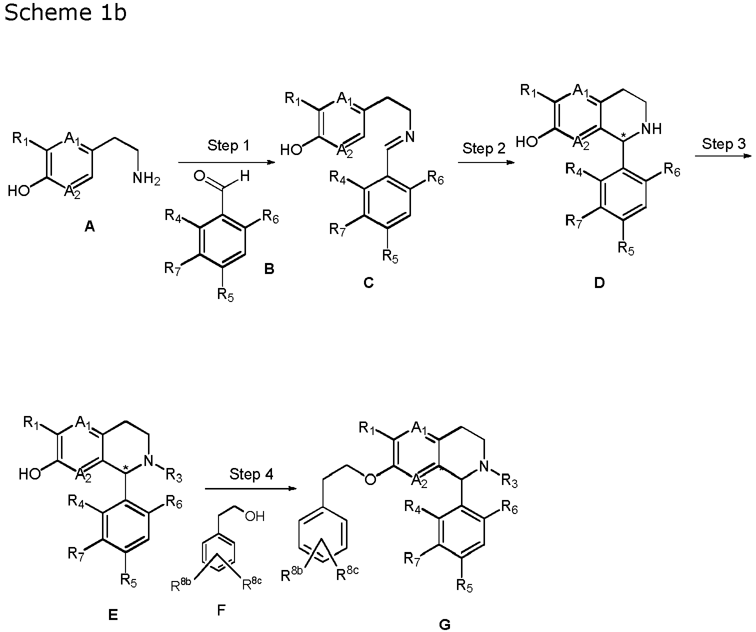

### Schemes 1 and 1b

Steps 1-2 involve a well-known Pictet-Spengler reaction [A. Yokohama et al., Journal of Organic Chemistry 1999, 64, 611-617*;* R. Gitto et al., Journal of Medicinal Chemistry 2003, 46, 197-200*]* where arylethylamines A are condensed with different substituted benzaldehydes B to give the corresponding imines C which upon treatment with refluxing trifluoroacetic acid undergo intramolecular cyclization to afford tetrahydroisoquinolines D as racemic mixtures. The Bischler-Napieralski reaction [J. E. De Los Angeles. Journal of Medicinal Chemistry 1996, 39, 3701-3711*;* G. Fodor et al., Angewandte Chemie Int. Ed. 1972, 11, 919-920*]* is alternatively used to synthesize tetrahydroisoquinolines D bearing an electron-withdrawing group in the R¹ position. At Step 3, the R³ substituent is introduced by means of different synthetic strategies well known in the art. At Step 4, ether G is prepared from phenol E by means of a Mitsunobu reaction (reagent F) [G. Liu. et al., Journal of Medicinal Chemistry 2007, 50, 3086-3100] under suitable conditions well known in the art. R⁹ is the protected version of R⁸ in case R⁸ contains substituents in need of protection during step 4.

Some of the compounds according to the present invention require an alternative synthetic strategy from that described in the Schemes 1 and 1b. These compounds might be prepared according to Scheme 2 described below.

### Scheme 2

At step 1, phenol A is protected using a suitable phenol protecting group PG₁, where PG₁ may be a benzyl group. Steps 2-3 involve a modified Hiyama coupling of compound B with 2-(trimethylsilyl)acetonitrile followed by a Pd-catalysed hydrogenation of the corresponding nitrile C to afford amine D (as set out in WO 2011/005636, page 37). At step 4, amine D reacts with acid E under suitable coupling conditions to give amide F. Introduction of substituent R₉ (reagent G) in step 5 is carried out by means of a Mitsunobu reaction [G. Liu. et al., Journal of Medicinal Chemistry 2007, 50, 3086-3100] to give amide H. Steps 6-7 involve a well-known Bischler-Napieralski reaction [J. E. De Los Angeles. Journal of Medicinal Chemistry 1996, 39, 3701-3711*;* G. Fodor et al., Angewandte Chemie Int. Ed. 1972, 11, 919-920] which is used to synthesize tetrahydroisoquinolines J. At Step 8, the R³ substituent is introduced by means of different synthetic strategies well known in the art. At step 9, R⁹ which is the protected version of R⁸ is transformed into R⁸ by means of well-known synthetic procedures.

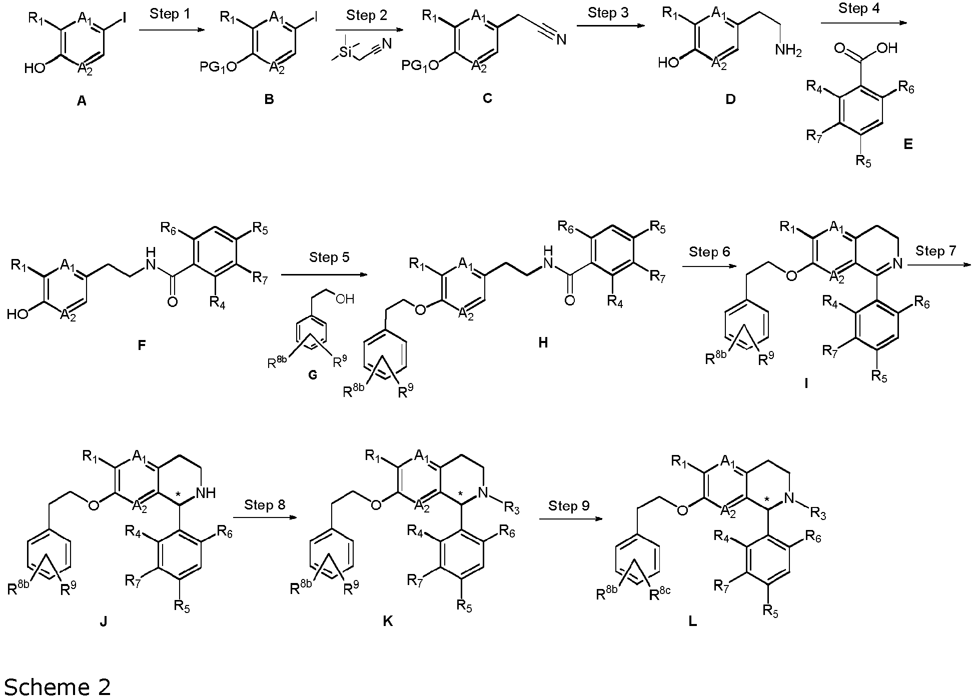

When R⁹-OH or R⁸-OH (in Scheme 1b) is 2-(4-methoxy-3-nitrophenyl) ethanol, it may be prepared according to Scheme 3 below:

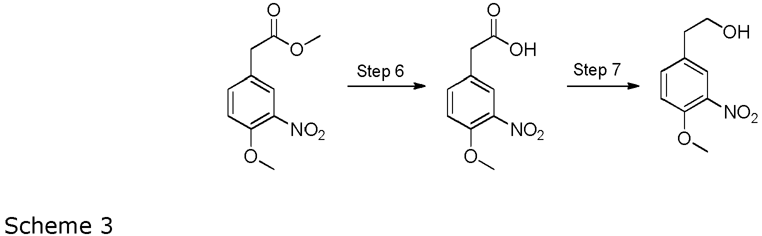

### Scheme 3

### Step 6 - Synthesis of 2-(4-methoxy-3-nitrophenyl) acetic acid

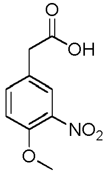

Over a solution of methyl 2-(4-methoxy-3-nitrophenyl) acetate (0.23 g, 1 mmol) in methanol (5 mL) and tetrahydrofuran (15 mL), lithium hydroxide (0.05 g, 2 mmol) was added followed by the addition of water (2.5 mL). The solution was kept under stirring at room temperature for 1h. The organic solvents were evaporated under vacuo, the aqueous residue was made acid by addition of 2N HCl and the product was extracted with ethyl acetate. The organic layer was dried over MgSO₄, filtrated and concentrated in vacuo to yield the crude product as a yellow solid (0.21 g, 98% yield). ¹H NMR (400 MHz, CDCl₃) δ ppm 7.80 (d, *J*=2.3 Hz, 1H), 7.47 (dd, *J*=8.6, 2.3 Hz, 1H), 7.07 (d, *J*=8.6 Hz, 1H), 3.96 (s, 3H), 3.66 (s, 2H).

### Step 7 - Synthesis of 2-(4-methoxy-3-nitrophenyl)ethanol

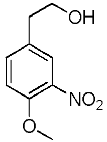

Borane-dimethylsulfide complex 2.0 M in THF (1.18 mL, 2.4 mmol) was added dropwise to a solution of 2-(4-methoxy-3-nitrophenyl) acetic acid (0.20 g, 0.9 mmol) in dry tetrahydrofuran (2 mL) at room temperature. The resulting solution was gradually warmed to 45°C and stirred for 60 min at this temperature. After this time the mixture was cooled to room temperature and excess borane was quenched by slow addition of methanol. The mixture was diluted with saturated NaHCO₃ and extracted with ethyl acetate. The organic layer was washed with brine, dried over MgSO₄, filtrated and concentrated in vacuo. The residue was purified by reverse phase chromatography (acetonitrile/water 0-100% gradient) to yield the title product as a light yellow solid (0.16 g, 86% yield). ¹H NMR (400 MHz, CDCl₃) δ ppm 7.73 (d, *J*=2.2 Hz, 1H), 7.42 (dd, *J*=8.6, 2.3 Hz, 1H), 7.03 (d, *J*=8.6 Hz, 1H), 3.94 (s, 3H), 3.87 (t, *J*=6.4 Hz, 2H), 2.86 (t, *J*=6.4 Hz, 2H).

When R⁹-OH is ethyl (4-(2-hydroxyethyl)-2-methoxyphenyl) carbamate it may be prepared according to Scheme 4 below:

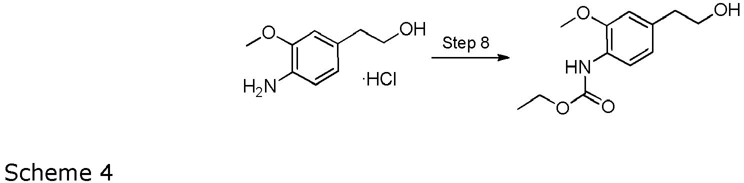

### Scheme 4

### Step 8 - Synthesis of ethyl (4-(2-hydroxyethyl)-2-methoxyphenyl) carbamate

To a cooled mixture (0°C) of 2-(4-amino-3-methoxyphenyl) ethanol hydrochloride (0.20 g, 0.93 mmol) in pyridine (1.4 mL) was added dropwise ethyl chloroformate (0.14 mL, 1.40 mmol). The resulting mixture was stirred at room temperature for 2 hours and at 60°C for 12 additional hours. The reaction mixture was diluted with water and the product extracted with ethyl acetate. The organic layer was dried over MgSO₄, filtrated and concentrated in vacuo. The crude product was purified by reverse phase chromatography (acetonitrile/water 0-100% gradient) to yield the pure product (0.16 g, 72% yield). ¹H NMR (400 MHz, CDCl₃) δ ppm 7.99 (d, *J*=8.1 Hz, 1H), 7.14 (bs, 1H), 6.81 (d, *J*=8.3 Hz, 1H), 6.72 (s, 1H), 4.22 (q, *J*=7.1 Hz, 2H), 3.81-3.85 (m, 5H), 2.82 (t, *J*=6.5 Hz, 2H), 1.31 (t, *J*=7.1 Hz, 3H).

When R⁹-OH is 2-(3-(benzyloxy)-4-methoxyphenyl)-ethanol it may be prepared according to Scheme 5 below:

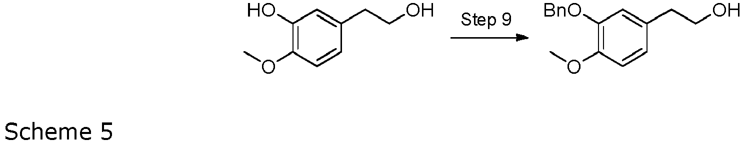

### Scheme 5

### Step 9 - Synthesis of 2-(3-(benzyloxy)-4-methoxyphenyl) ethanol

To a solution of 5-(2-hydroxyethyl)-2-methoxyphenol (0.13 g, 0.71 mmol) and potassium carbonate (0.20 g, 1.41 mmol) in anhydrous DMF (3 mL) was added benzyl bromide (0.09 mL, 0.71 mmol). The resulting mixture was stirred at 50 °C. After 4 hours, the mixture was diluted with water and the product extracted with ethyl acetate. The organic layer was dried over MgSO₄, filtrated and concentrated under reduced pressure. The crude product was purified by reverse phase chromatography (acetonitrile/water 0-100% gradient) to yield the pure product as a beige solid (0.12 g, 65% yield). ¹H NMR (400 MHz, CDCl₃) δ ppm 7.43-7.45 (m, 2H), 7.28-7.38 (m, 3H), 6.85 (d, *J*=8.7 Hz, 1H), 6.77-6.79 (m, 2H), 5.15 (s, 2H), 3.87 (s, 3H), 3.77 (t, *J*=6.4 Hz, 2H), 2.75 (t, *J*=6.4 Hz, 2H).

### Examples

### Example 1 - Synthesis of compound 6: 7-(3-amino-4-methoxyphenethoxy)-1-(2,4-dimethylphenyl)-N-ethyl-6-methoxy-3,4-dihydroisoquinoline-2(1H)-carboxamide

### Step 1 - Synthesis of 4-(2-((2,4-dimethylbenzylidene)amino)ethyl)-2-methoxyphenol

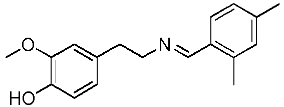

To a solution of 4-(2-aminoethyl)-2-methoxyphenol hydrochloride (0.49 g, 2.4 mmol) in methanol (9 mL), triethylamine (2.6 mL, 18.9 mmol) and activated molecular sieves were added followed by the addition of 2,4-dimethylbenzaldehyde (0.35 g, 2.4 mmol) in toluene (15 mL). The reaction was stirred at reflux. After 2 h, the reaction mixture was dried over anhydrous MgSO₄, diluted with dichloromethane, filtered and concentrated under vacuo to give the crude product which was immediately used without purification as starting material in step 2.

### Step 2 - Synthesis of 1-(2,4-dimethylphenyl)-6-methoxy-1,2,3,4-tetrahydroisoquinolin-7-ol

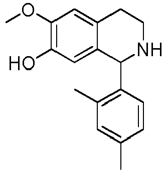

4-(2-((2,4-dimethylbenzylidene)amino)ethyl)-2-methoxyphenol was mixed with trifluoroacetic acid (25 mL). The reaction was stirred at reflux for 3 h. The reaction mixture was diluted with water and extracted with dichloromethane (x3). The combined organic layers were dried over anhydrous MgSO₄, filtered and concentrated under vacuo. The crude product was purified by reverse phase chromatography (acetonitrile+0.1% TFA/water+0.1% TFA 0-100% gradient) to give the title product as a beige solid (TFA salt) which was converted to the free amine by basic treatment (washing twice with 2N NaOH). The free amine was obtained as a beige solid (0.37 g, 55% yield). ¹H NMR (400 MHz, CD₃OD) δ ppm 7.20 (s, 1H), 7.09 (d, *J*=7.8 Hz, 1H), 6.95 (d, *J*=8.0 Hz, 1H), 6.84 (s, 1H), 6.11 (s, 1H), 5.80 (s, 1H), 3.87 (s, 3H), 3.48-3.59 (m, 2H), 3.20-3.28 (m, 1H), 3.04-3.11 (m, 1H), 2.47 (s, 3H), 2.34 (s, 3H).

### Step 3 - Synthesis of 1-(2,4-dimethylphenyl)-N-ethyl-7-hydroxy-6-methoxy-3,4-dihydroisoquinoline-2(1H)-carboxamide

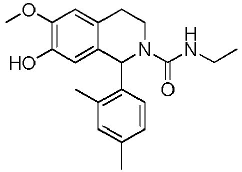

To a solution of ethylamine hydrochloride (0.43 g, 5.2 mmol) in dry *N,N-*dimethylformamide (3.9 mL) was added triethylamine (1.45 mL, 10.4 mmol). The mixture was stirred at room temperature for 10 minutes, after which time was added 1,1'-carbonyldiimidazole (0.70 g, 3.9 mmol). The mixture was stirred at room temperature for 1 h, after which time was added 1-(2,4-dimethylphenyl)-6-methoxy-1,2,3,4-tetrahydroisoquinolin-7-ol (0.37 g, 1.3 mmol) dissolved in dry *N,N-*dimethylformamide (6.5 mL). The reaction mixture was stirred at room temperature. After 3h, the reaction mixture was partitioned between ethyl acetate and water. The organic layer was washed with brine, dried over anhydrous MgSO₄, filtered and concentrated under vacuo. The crude product was purified by reverse phase chromatography (acetonitrile/water 0-100% gradient) to yield the title product as a beige solid (0.44 g, 95% yield). ¹H NMR (400 MHz, CD₃OD) δ ppm 7.00 (s, 1H), 6.82 (d, *J*=8.2 Hz, 1H), 6.72 (s, 1H), 6.53 (d, *J*=7.9 Hz, 1H), 6.38 (s, 1H), 6.35 (s, 1H), 3.85 (s, 3H), 3.72 (ddd, *J*=14.4, 5.8, 1.6 Hz, 2H), 3.13-3.24 (m, 3H), 2.89-2.98 (m, 1H), 2.58 (dd, *J*=16.3, 3.9 Hz, 1H), 2.40 (s, 3H), 2.25 (s, 3H), 1.10 (t, *J*=7.2 Hz, 3H).

### Step 4 - Synthesis of 1-(2,4-dimethylphenyl)-N-ethyl-6-methoxy-7-(4-methoxy-3-nitrophenethoxy)-3,4-dihydroisoquinoline-2(1H)-carboxamide

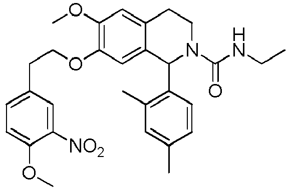

2-(4-methoxy-3-nitrophenyl)-ethanol (0.08 g, 0.41 mmol) was dissolved in dry toluene (1.6 mL). 1-(2,4-dimethylphenyl)-N-ethyl-7-hydroxy-6-methoxy-3,4-dihydroisoquinoline-2(1H)-carboxamide (0.14 g, 0.41 mmol) and triphenylphosphine (0.14 g, 0.53 mmol) were added. The mixture was heated at reflux. After 5 minutes diisopropylazodicarboxylate (0.11 mL, 0.53 mmol) was slowly added and the reaction mixture was stirred at reflux for 2 additional hours. The solvent was evaporated and the residue was immediately purified by reverse phase chromatography (acetonitrile/water 0-100% gradient) to yield the title product as a yellow solid (0.17 g, 77% yield). ¹H NMR (400 MHz, CD₃OD) δ ppm 7.75 (d, *J*=2.2 Hz, 1H), 7.48 (dd, *J*=8.6, 2.3 Hz, 1H), 7.14 (d, *J*=8.6 Hz, 1H), 7.00 (s, 1H), 6.80 (d, *J*=7.4 Hz, 1H), 6.75 (s, 1H), 6.44-6.47 (m, 3H), 4.00-4.12 (m, 2H), 3.90 (s, 3H), 3.80 (s, 3H), 3.71 (dd, *J*=14.6, 5.9 Hz, 1H), 3.13-3.26 (m, 3H), 2.90-2.99 (m, 3H), 2.59 (dd, *J*=16.4, 4.0 Hz, 1H), 2.39 (s, 3H), 2.24 (s, 3H), 1.10 (t, *J*=7.2 Hz, 3H).

### Step 5 - Synthesis of 7-(3-amino-4-methoxyphenethoxy)-1-(2,4-dimethylphenyl)-N-ethyl-6-methoxy-3,4-dihydroisoquinoline-2(1H)-carboxamide

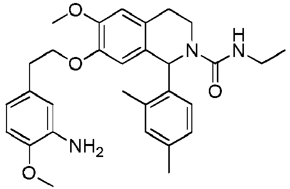

Sodium dithionite (0.23 g, 1.10 mmol) was added over a solution of 1-(2,4-dimethylphenyl)-N-ethyl-6-methoxy-7-(4-methoxy-3-nitrophenethoxy)-3,4-dihydroisoquinoline-2(1H)-carboxamide (0.17 g, 0.32 mmol) in a mixture DMF/water 9:1 (2.2 mL). The temperature was set up to 50°C and the reaction mixture was stirred at this temperature for 24 hours. After this time, an additional amount of sodium dithionite (0.23 g, 1.10 mmol) was added and the mixture stirred for 24 extra hours. The reaction mixture was diluted with water and the product extracted with ethyl acetate. The organic layer was dried over anhydrous MgSO₄, filtered and concentrated under vacuo. The crude product was purified by reverse phase chromatography (acetonitrile/water 0-100% gradient) to yield the title product as a white solid (0.07 g, 41% yield). ¹H NMR (400 MHz, CD₃OD) δ ppm 7.01 (s, 1H), 6.82 (d, *J*=8.0 Hz, 1H), 6.76 (s, 1H), 6.71 (d, *J*=8.2 Hz, 1H), 6.63 (d, *J*=2.1 Hz, 1H), 6.53 (dd, *J*=8.2, 2.1 Hz, 1H), 6.49 (d, *J*=7.8 Hz, 1H), 6.43 (s, 2H), 3.92-4.05 (m, 2H), 3.82 (s, 3H), 3.79 (s, 3H), 3.72 (dd, *J*=14.2, 5.6 Hz, 1H), 3.13-3.26 (m, 3H), 2.91-2.99 (m, 1H), 2.83 (t, *J*=7.2 Hz, 2H), 2.59 (dd, *J*=16.5, 4.0 Hz, 1H), 2.40 (s, 3H), 2.25 (s, 3H), 1.10 (t, *J*=7.2 Hz, 3H).

In addition to compound 6, compounds 4, 7, 10-13 and 15-28 may also be prepared according to scheme 1 and the methodology of example 1 but using the appropriately substituted reagents. Compound 8 may be prepared according to scheme 1b.

### Example 2 - Synthesis of compound 9: 7-(4-amino-3-methoxyphenethoxy)-1-(2,4-dimethylphenyl)-N-ethyl-6-methoxy-3,4-dihydroisoquinoline-2(1H)-carboxamide Steps 1, 2 and 3 are described in Example 1.

### Step 4 - Synthesis of ethyl (4-(2-((1-(2,4-dimethylphenyl)-2-(ethylcarbamoyl)-6-methoxy-1,2,3,4-tetrahydroisoquinolin-7-yl)oxy)ethyl)-2-methoxyphenyl)carbamate

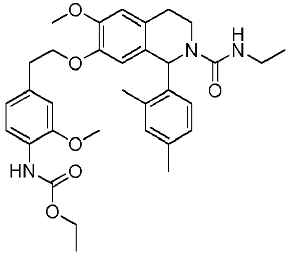

Ethyl (4-(2-hydroxyethyl)-2-methoxyphenyl) carbamate (0.10 g, 0.42 mmol) was dissolved in dry toluene (0.9 mL). 1-(2,4-dimethylphenyl)-N-ethyl-7-hydroxy-6-methoxy-3,4-dihydroisoquinoline-2(1H)-carboxamide (0.15 g, 0.42 mmol) and triphenylphosphine (0.14 g, 0.55 mmol) were added. The mixture was heated at reflux. After 5 minutes, diisopropylazodicarboxylate (0.11 mL, 0.55 mmol) was slowly added and the reaction mixture was stirred at reflux for 2 additional hours. The solvent was evaporated and the residue was immediately purified by reverse phase chromatography (acetonitrile/water 0-100% gradient) to yield the title product as a yellow solid (0.17 g, 70% yield). ¹H NMR (400 MHz, CDCl₃) δ ppm 7.94 (bs, 1H), 7.11 (s, 1H), 7.01 (s, 1H), 6.83 (d, *J*=8.0 Hz, 1H), 6.75-6.77 (m, 2H), 6.61-6.63 (m, 2H), 6.40-6.41 (m, 2H), 4.47 (t, *J*=5.2 Hz, 1H), 4.21 (q, *J*=7.1 Hz, 2H), 3.94-4.07 (m, 2H), 3.84 (s, 3H), 3.82 (s, 3H), 3.60 (dd, J=14.8, 5.3 Hz, 2H), 3.23-3.36 (m, 3H), 2.94-3.01 (m, 3H), 2.63 (d, *J*=16.0 Hz, 1H), 2.46 (s, 3H), 2.27 (s, 3H), 1.31 (t, *J*=7.1 Hz, 3H), 1.09 (t, *J*=7.2 Hz, 3H).

### Step 5 - Synthesis of 7-(4-amino-3-methoxyphenethoxy)-1-(2,4-dimethylphenyl)-N-ethyl-6-methoxy-3,4-dihydroisoquinoline-2(1H)-carboxamide

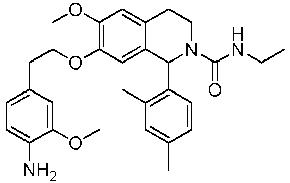

Ethyl (4-(2-((1-(2,4-dimethylphenyl)-2-(ethylcarbamoyl)-6-methoxy-1,2,3,4-tetrahydroisoquinolin-7-yl)oxy)ethyl)-2-methoxyphenyl)carbamate (0.17 g, 0.30 mmol) was dissolved in ethanol (6 mL). KOH 2M solution (13 mL) was added and the reaction mixture stirred at reflux for 16 h. The reaction mixture was diluted with water and the product extracted with ethyl acetate. The organic layer was dried over anhydrous MgSO₄, filtered and concentrated under vacuo. The crude product was purified by reverse phase chromatography (acetonitrile/water 0-100% gradient) followed by TLC preparative chromatography on silica gel (100% ethyl acetate) to give the title product as a beige solid (0.06 g, 39% yield). ¹H NMR (400 MHz, CD₃OD) δ ppm 7.04 (s, 1H), 6.85 (dd, *J*=7.7 Hz, 1H), 6.78-6.80 (m, 2H), 6.69 (d, *J*=7.8 Hz, 1H), 6.61 (dd, *J*=7.9, 1.6 Hz, 1H), 6.52 (d, *J*=7.8 Hz, 1H), 6.48 (s, 1H), 6.47 (s, 1H), 3.96-4.10 (m, 2H), 3.85 (s, 3H), 3.81 (s, 3H), 3.76 (dd, *J*=14.5, 5.8 Hz, 1H), 3.16-3.29 (m, 3H), 2.94-3.03 (m, 1H), 2.90 (t, *J*=6.9 Hz, 2H), 2.62 (dd, *J*=16.5, 3.7 Hz, 1H), 2.43 (s, 3H), 2.28 (s, 3H), 1.14 (t, *J*=7.2 Hz, 3H).

In addition to compound 9, compounds 14 and 29-46 may also be prepared according to scheme 1 and the methodology of example 2 but using the appropriately substituted reagents.

### Example 3 - Synthesis of compound 5: 1-(2,4-dimethylphenyl)-N-ethyl-7-(3-hydroxy-4-methoxyphenethoxy)-6-methoxy-3,4-dihydroisoquinoline-2(1H)-carboxamide Steps 1, 2 and 3 are described in Example 1.

### Step 4 - Synthesis of 7-(3-(benzyloxy)-4-methoxyphenethoxy)-1-(2,4-dimethylphenyl)-N-ethyl-6-methoxy-3,4-dihydroisoquinoline-2(1H)-carboxamide

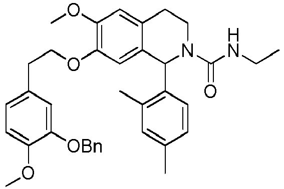

2-(3-(benzyloxy)-4-methoxyphenyl) ethanol (0.04 g, 0.16 mmol) was dissolved in dry toluene (0.6 mL). 1-(2,4-dimethylphenyl)-N-ethyl-7-hydroxy-6-methoxy-3,4-dihydroisoquinoline-2(1H)-carboxamide (0.06 g, 0.16 mmol) and triphenylphosphine (0.06 g, 0.21 mmol) were added. The mixture was heated at reflux. After 5 minutes, diisopropylazodicarboxylate (0.04 mL, 0.21 mmol) was slowly added and the reaction mixture was stirred at reflux for 2 additional hours. The solvent was evaporated and the residue was immediately purified by reverse phase chromatography (acetonitrile/water 0-100% gradient) to yield the title product as a beige solid (0.08 g, 78% yield). ¹H NMR (400 MHz, CDCl₃) δ ppm 7.40-7.43 (m, 2H), 7.24-7.34 (m, 3H), 7.01 (s, 1H), 6.78-6.86 (m, 3H), 6.72 (dd, *J*=8.1, 1.9 Hz, 1H), 6.63-6.67 (m, 2H), 6.42 (s, 1H), 6.37 (s, 1H), 5.09 (s, 2H), 3.87-3.99 (m, 2H), 3.84-3.85 (m, 6H), 3.58 (dd, *J*=14.5, 5.4 Hz, 1H), 3.24-3.38 (m, 3H), 2.98-3.03 (m, 1H), 2.92-2.95 (m, 2H), 2.68 (d, *J*=16.0 Hz, 1H), 2.46 (s, 3H), 2.27 (s, 3H), 1.09 (t, *J*=7.2 Hz, 3H).

### Step 5 - Synthesis of 1-(2,4-dimethylphenyl)-N-ethyl-7-(3-hydroxy-4-methoxyphenethoxy)-6-methoxy-3,4-dihydroisoquinoline-2(1H)-carboxamide

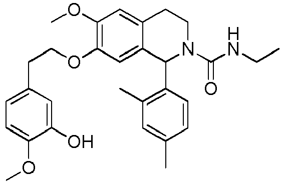

7-(3-(benzyloxy)-4-methoxyphenethoxy)-1-(2,4-dimethylphenyl)-N-ethyl-6-methoxy-3,4-dihydroisoquinoline-2(1H)-carboxamide (0.08 g, 0.13 mmol) was shaken in a mixture toluene/TFA 1:1 at room temperature. After 3 days, the reaction mixture was diluted with water, made basic by addition of 2N NaOH and the product extracted with ethyl acetate. The organic layer was washed 5 times with aq. 2N NaOH, dried over anhydrous MgSO₄, filtered and concentrated under vacuo. The crude product was purified by reverse phase chromatography (acetonitrile/water 0-100% gradient) to yield the title product as a beige solid (0.02 g, 32% yield). ¹H NMR (CD₃OD, 400 MHz) δ ppm 7.01 (s, 1H), 6.77-6.84 (m, 3H), 6.69 (d, *J*=2.1 Hz, 1H), 6.63 (dd, *J*=8.2, 2.1 Hz, 1H), 6.49 (d, *J*=7.9 Hz, 1H), 6.45 (s, 1H), 6.43 (s, 1H), 3.93-4.06 (m, 2H), 3.82 (s, 3H), 3.80 (s, 3H), 3.73 (dd, J=14.4, 5.9 Hz, 1H), 3.14-3.26 (m, 3H), 2.91-2.99 (m, 1H), 2.85 (t, *J*=7.0 Hz, 2H), 2.59 (dd, *J*=16.2, 4.1 Hz, 1H), 2.40 (s, 3H), 2.25 (s, 3H), 1.10 (t, J=7.2 Hz, 3H).

In addition to compound 5, compounds 1-3 may also be prepared according to scheme 1 and the methodology of example 3, but using the appropriately substituted reagents.

### Example 4 - activity in tumor cell lines

- Cell line #1: A549. Lung carcinoma cell line bearing KRas^{G125} oncogenic mutation
- Cell line #2: H358. non-small cell lung cancer line bearing KRas^{G12C} oncogenic mutation
- Cell line #3: PANC-1. epithelioid carcinoma of the pancreas cell line bearing KRas^{G12D} oncogenic mutation
- Cell line #4: RPMI. myeloma cell line bearing KRas^{G12A} oncogenic mutation
Cell lines were cultured in DMEM or RPMI-1640 supplemented with FBS 10%. In order to assess the antiproliferative effect of compounds, cells were seeded at a density of 1.8×10³, 6.2×10³, 7.8×10³, 21×10³ and 2×10³ cells/cm², respectively, in tissue culture microplates and were incubated in humidified atmosphere at 5% CO₂. 24h later, compounds dissolved in DMSO 100% were added for different final concentrations ranging between 0.1 and 50 µM for a final DMSO concentration of 0.5% and the plates were incubated for another 72 h. After incubation, proliferation was quantified using CellTiter 96^{®} Aqueous Non-Radioactive Cell Proliferation Assay-MTS (Promega #G5421) following manufacturer instructions. Amount of 490nm absorbance is directly proportional to the number of living cells. Absorbance was recorded with a BMG Fluostar Optima Microplate Reader and normalized to control with vehicle.

### IC50 values (µM): cell proliferation inhibition

| Compound | H358 | Panc-1 | RPMI | A549 |
|---|---|---|---|---|
| 1 | 1.5 | 2 | 1.5 | 2 |
| 2 | 0.4 | 0.3 | 0.2 | 1 |
| 3 | >2 | >2 | >2 | >2 |
| 4 | 0.2 | 0.4 | 0.3 | 0.9 |
| 5 | 0.1 | 0.2 | 0.1 | 0.7 |
| 6 | 0.2 | 0.2 | 0.1 | 0.6 |
| 7 | 0.9 | 0.8 | 0.6 | 0.6 |
| 8 | 0.2 | 0.2 | 0.2 | 0.9 |
| 9 | 0.4 | 0.3 | 0.2 | 1 |
| 10 | 0.3 | 0.2 | 0.1 | 0.9 |
| 11 | 0.2 | 0.2 | 0.1 | 0.8 |
| 12 | 0.2 | 0.1 | 0.1 | 0.9 |
| 13 | 0.3 | 0.3 | 0.1 | 1 |
| 14 | 0.7 | 0.5 | 0.4 | 1.6 |
| 15 | 0.3 | 0.3 | 0.1 | 1 |
| 16 | 0.3 | 0.2 | 0.1 | 1 |
| 17 | 0.5 | 0.4 | 0.2 | 1 |
| 18 | 0.3 | 0.2 | 0.1 | 1 |
| 19 | 0.5 | 0.3 | 0.5 | 1.5 |
| 20 | 0.4 | 0.4 | 0.2 | 1 |
| 21 | 0.2 | 0.2 | 0.1 | 0.9 |
| 22 | 0.7 | 0.6 | 0.5 | >2 |
| 23 | 0.1 | 0.1 | 0.1 | 0.7 |
| 24 | 0.2 | 0.2 | 0.1 | 1 |
| 25 | 0.6 | 0.5 | 0.3 | >2 |
| 26 | 0.4 | 0.5 | 0.2 | 1 |
| 27 | 0.3 | 0.3 | 0.1 | 0.9 |
| 28 | 0.3 | 0.2 | 0.1 | 0.8 |
| 29 | 0.5 | 0.4 | 0.2 | 1.5 |
| 30 | 0.4 | 0.5 | 0.3 | 1 |
| 31 | 0.5 | 0.4 | 0.1 | >2 |
| 32 | 0.3 | 0.2 | 0.1 | 1 |
| 33 | 0.8 | 0.6 | 0.7 | >2 |
| 34 | 0.5 | 0.4 | 0.1 | 1.2 |
| 35 | 0.4 | 0.4 | 0.3 | 1 |
| 36 | 0.9 | 0.7 | 0.5 | 1.7 |
| 37 | 0.4 | 0.4 | 0.1 | 1.5 |
| 38 | 0.5 | 0.4 | 0.2 | 1.5 |
| 39 | 0.6 | 0.6 | 0.3 | >2 |
| 40 | 0.3 | 0.3 | 0.1 | 1 |
| 41 | 0.9 | 0.7 | 0.9 | >2 |
| 42 | 0.3 | 0.3 | 0.1 | 1 |
| 43 | 0.3 | 0.2 | 0.1 | 1 |
| 44 | 0.8 | 0.5 | 0.3 | >2 |
| 45 | 0.6 | 0.3 | 0.2 | 1 |
| 46 | 0.5 | 0.3 | 0.1 | 1 |
| 47 | 0.6 | >1 | 0.4 | >2 |
| 48 | 0.1 | 0.5 | 0.04 | >1 |
| 49 | >0.8 | 1.5 | >1 | >2 |
| 50 | 0.5 | 0.2 | 0.15 | >2 |
| 51 | 0.15 | 0.07 | >1 | 0.2 |
| 52 | 1 | 1 | 0.2 | >2 |
| Reference Example 1 | 0.3 | | | |

Data shown for compounds 1-23 and 47-52 and Reference Example 1 are the median from experimental results. Data shown for compounds 24-46 are based on estimations and/or preliminary experimental results.

### Example 5: Efficacy testing in 3D viability assay for NIH-H358 cell line

The protocol to perform 3D CellTiter-GloTM cell viability assay is as follows:
Day -1: Cell plating
   - Adjust cell concentrations to 1×105 cells/ml with respective medium. (Cell concentration is adjusted according to data base or density optimization assay). Mix 3.5 mL of cell suspension 6.5 mL of 1% methylcellulose. Mix and wait for bubbles to disperse before pipetting. This step yields 10 ml of cell suspension in 0.65% methylcellulose solution. Add 99.5 µL cell suspensions to 96-well plates according to plate map with final cell density.
   - Two duplicate plates will be set up. One is for day 0 reading (T0) and the other will be cultured in incubator for reading at the end point.
   - Incubate the plates overnight in humidified incubator at 37° C with 5% CO2.
Day 0: T0 plate reading and compound treatment
   - Take T0 plate, add 0.5 µL culture medium to each well for T0 reading.
   - Add 100 µl CellTiter-Glo^{®} Reagent to each well.
   - Mix contents for 2 minutes on an orbital shaker to facilitate cell lysis.
   - Allow the plate to incubate at room temperature for 10 minutes to stabilize luminescent signal.
   - Record luminescence using EnVision Multi Label Reader.
   - Dilute the test articles at the concentration indicated at Test Articles Dilution. Add 0.5 µL of each 200X compound working solutions according to plate inoculation map.
Day 7: Plate reading of 7 days' compound treatment
   - Add 100 µL CellTiter-Glo^{®} Reagent to each well.
   - Mix contents for 2 minutes on an orbital shaker to facilitate cell lysis.
   - Allow the plate to incubate at room temperature for 10 minutes to stabilize luminescent signal.
   - Record luminescence using EnVision Multi Label Reader.

### Results:

| Compound | IC50 (µM) |
|---|---|
| 1 | >1 |
| 2 | 0.299 |
| 3 | >1 |
| 4 | 0.556 |
| 5 | 0.533 |
| 6 | 0.125 |
| 7 | 0.531 |
| 8 | >3 |
| 9 | 0.645 |
| 10 | 0.360 |
| 11 | 0.413 |
| 13 | 0.147 |
| 47 | 0.963 |
| 48 | 0.371 |
| 50 | 0.767 |
| 51 | 0.178 |
| 52 | >1 |

### Example 6. Efficacy in xenograft studies.

Evaluation of the efficacy of compounds 6, 11 and 13 in the treatment of subcutaneous NCI-H358 Human Lung Cancer Model in NCr nude mice.

### Experimental design

The treatment with compound 6 started when the tumors size reached 170 mm³ (study 1). This value was 190 mm³ in the case of compounds 11 and 13 (study 2). Each group administration and the number of animals in each study are shown in the following tables:

### Study 1

| **Group** | **Number of animals** | **Treatment** | **Dose (mg / kg)** | **Dosing** | **Schedule** |
|---|---|---|---|---|---|
| 1 | 8 | Vehicle control | - | peroral (p.o.) | BID × 29 days |
| 2 | 8 | compound 6 | 20 | peroral | BID × 29 days |
| 3 | 8 | Cisplatin | 3 | intraperitoneal (i.p.) | BIW × 29 days |

| | | | | | |
|---|---|---|---|---|---|
| Dosing volume: 10 ml/Kg | | | | | |

### Study 2

| **Group** | **Number of animals** | **Treatment** | **Dose (mg / kg)** | **Dosing** | **Schedule** |
|---|---|---|---|---|---|
| 1 | 8 | Vehicle control | - | peroral | BID × 27 days |
| 2 | 8 | compound 11 | 20 | peroral | BID × 27 days |
| 3 | 8 | compound 13 | 20 | peroral | BID × 27 days |

| | | | | | |
|---|---|---|---|---|---|
| Dosing volume: 10 ml/Kg | | | | | |

Studies endpoints: The major endpoints of the study included the following:
Tumor growth inhibition (TGI): TGI(%) is an indication of antitumor effectiveness, and it is expressed as: TGI (%)=100 × (1-T/C). T and C were the mean tumor volume of the treated and control groups, respectively, on a given day.

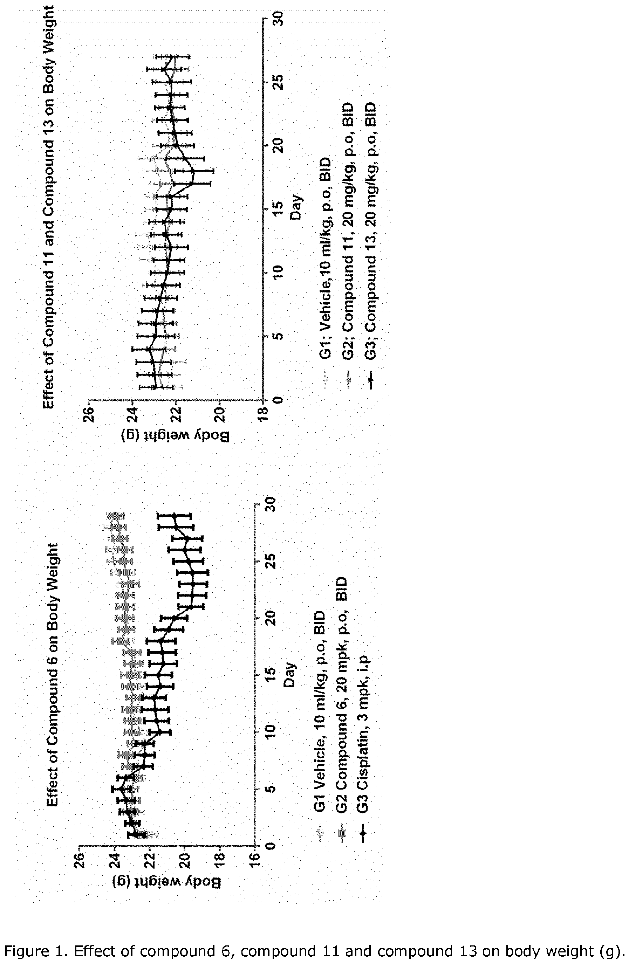

The results of the body weight changes in the tumor bearing mice are shown in Figure 1 (p.o. is peroral; i.p. is intraperitoneal). The body weight of the animals at the end of the treatment with the three compounds was only slightly below the body weight of the respective vehicle control groups. The results suggest that the mice bearing the subcutaneous NCI-H358 human lung cancer xenograft model tolerate 20 mg/kg BID of the compounds 6, 11 and 13.

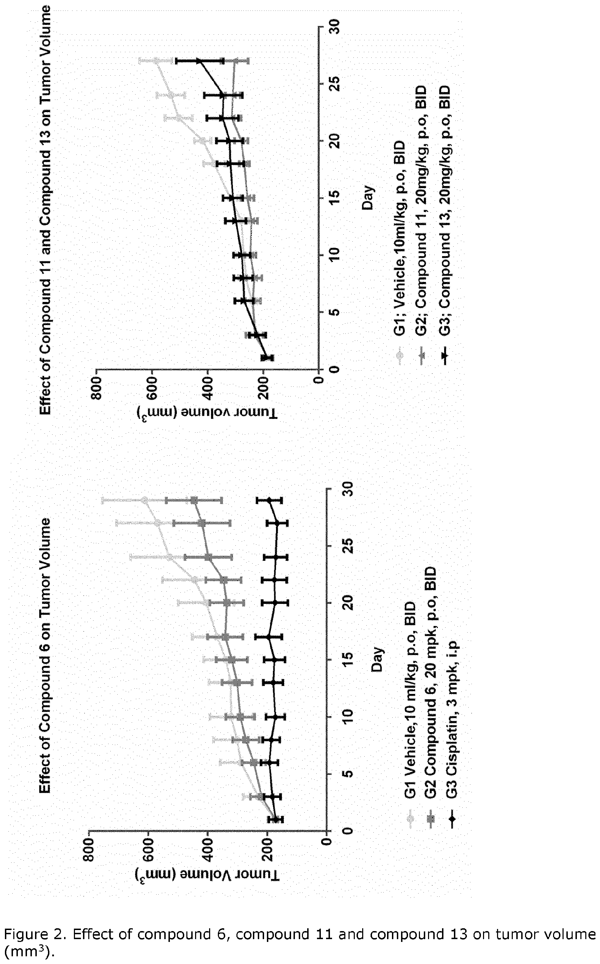

The tumor growth curves of the different groups are shown in Figure 2 (p.o. is peroral; i.p. is intraperitoneal). The TGI values (%) found after the treatment with the compounds 6, 11 and 13 were 27.09%, 48.22% and 26.99%.

The test compounds 6, 11 and 13 demonstrated significant oral anti-tumor activities in subcutaneous NCI-H358 human lung cancer xenograft model, and 20 mg/kg BID dose is safe for the bearing mice.

## Claims

1. A compound of Formula I, enantiomers and pharmaceutically acceptable salts thereof: 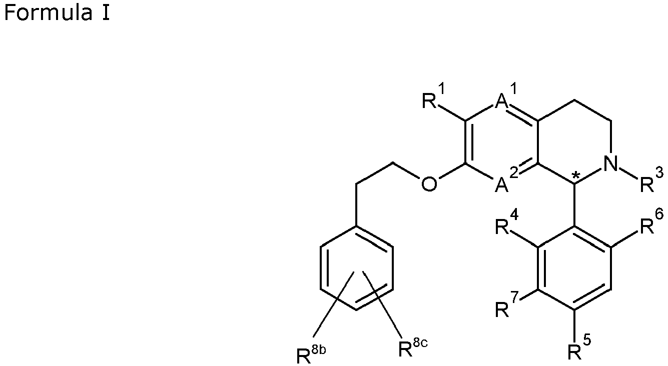 wherein
A¹ is selected from CR² and N;
A² is selected from CH and N;
R¹ is OR^{x},
R^{x} is C₁₋₄ alkyl, or H;
R² is H, C₁₋₄ alkyl, C₁₋₄ haloalkyl, halogen, OC₁₋₄ alkyl, OC₁₋₄ haloalkyl, OH or NH₂;
R³ is -(CH₂)ₙ³-C(Y³)-(X³)ₘ³-(CH2)ₖ³-R^{3a},
n³ is an integer in the range of 0 to 2,
Y³ is O,
X³ is NH, or O,
m³ is 0 or 1,
k³ is 0 or 1,
R^{3a} is C₁₋₄ alkyl, C₁₋₄ haloalkyl, or Het³,
Het³ is oxazolyl, isoxazolyl, thiazolyl, isothiazolyl, or pyridinyl;
R⁴ is halogen, C₁₋₄ alkyl, C₁₋₄ haloalkyl, OC₁₋₄ alkyl, or OC₁₋₄ haloalkyl;
R⁵ is H, halogen, OC₁₋₄ alkyl, OH, C₁₋₄ alkyl, C₁₋₄ haloalkyl, or OC₁₋₄ haloalkyl;
R⁶ is H, OH, or halogen;
R⁷ is H, halogen, OH, or C₁₋₄ alkyl;
R^{8b} and R^{8c} are independently selected from the group consisting of OH, C₁₋₄ alkyl, NH₂, NO₂, OC₁₋₄ alkyl, and halogen.

2. The compound according to any preceding claim, having Formula II, enantiomers or pharmaceutically acceptable salts thereof: 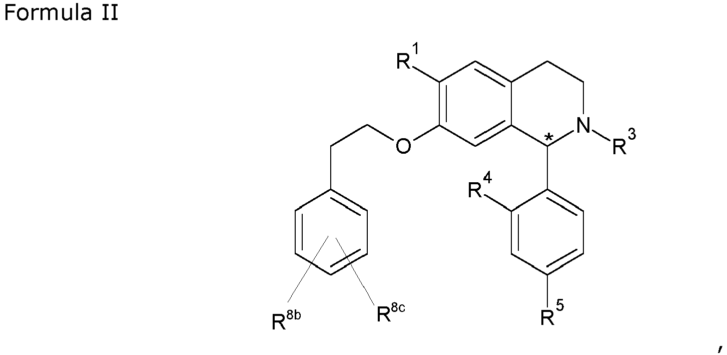 wherein R¹, R³, R⁴, R⁵, R^{8b}, and R^{8c} are as defined in any preceding claim.

3. The compound according to claim 1 or 2, wherein the phenyl ring is a phen-1-yl substituted in positions 3 and 4.

4. The compound according to any one of claims 1 to 3, wherein X³ is NH.

5. The compound according to claim 4, wherein n³ is 0 and m³ is 1.

6. The compound according to any one of claims 4 or 5, wherein R^{3a} is oxazolyl, methyl, or trifluoromethyl.

7. The compound according to any one of claims 4 to 6, wherein k³ is 1.

8. The compound according to any one of claims 1 to 7, wherein A¹ is CH.

9. The compound according to any one of claims 1 to 8, wherein R⁶ and R⁷ are H.

10. The compound according to any one of claims 1 to 9, wherein R⁵ is F, Cl, CF₃, CH₃, or OCH₃.

11. The compound according to any one of the preceding claims which is selected from:

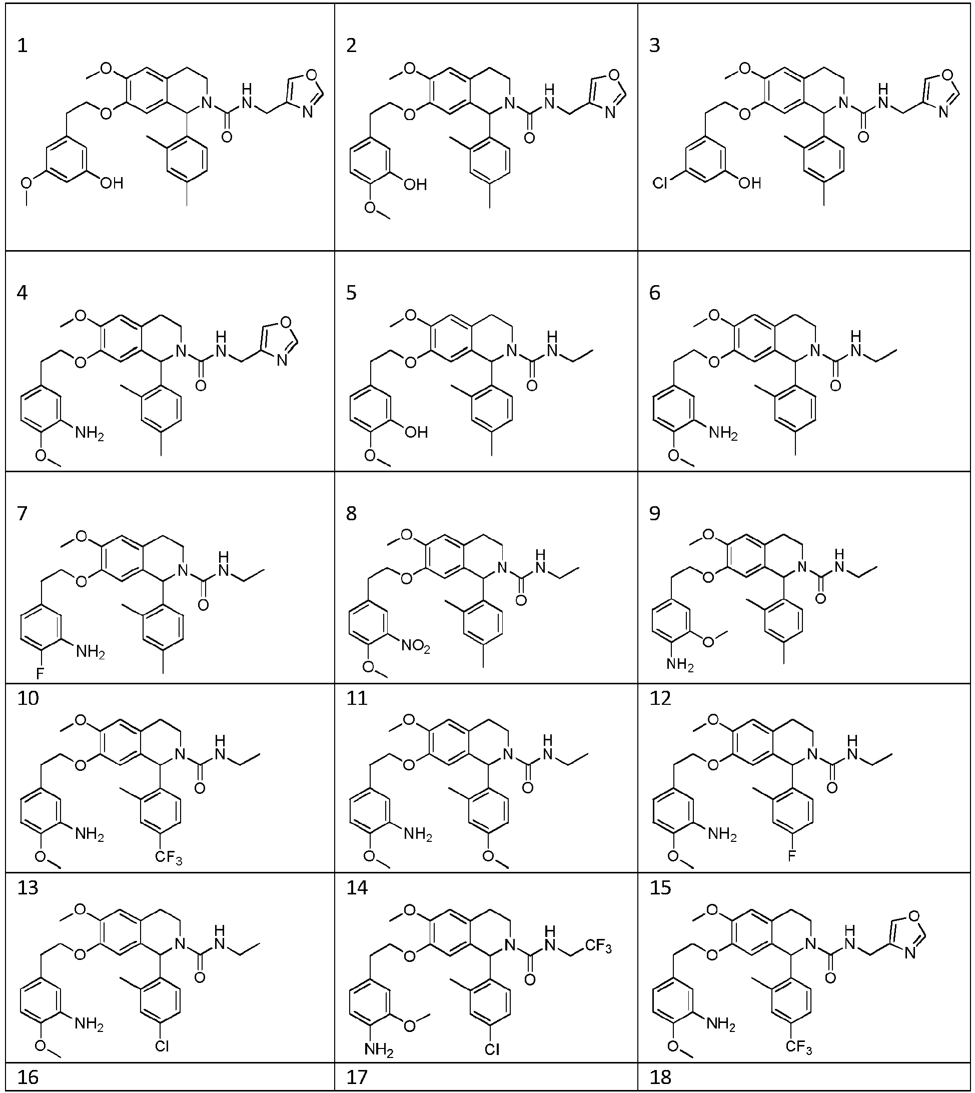

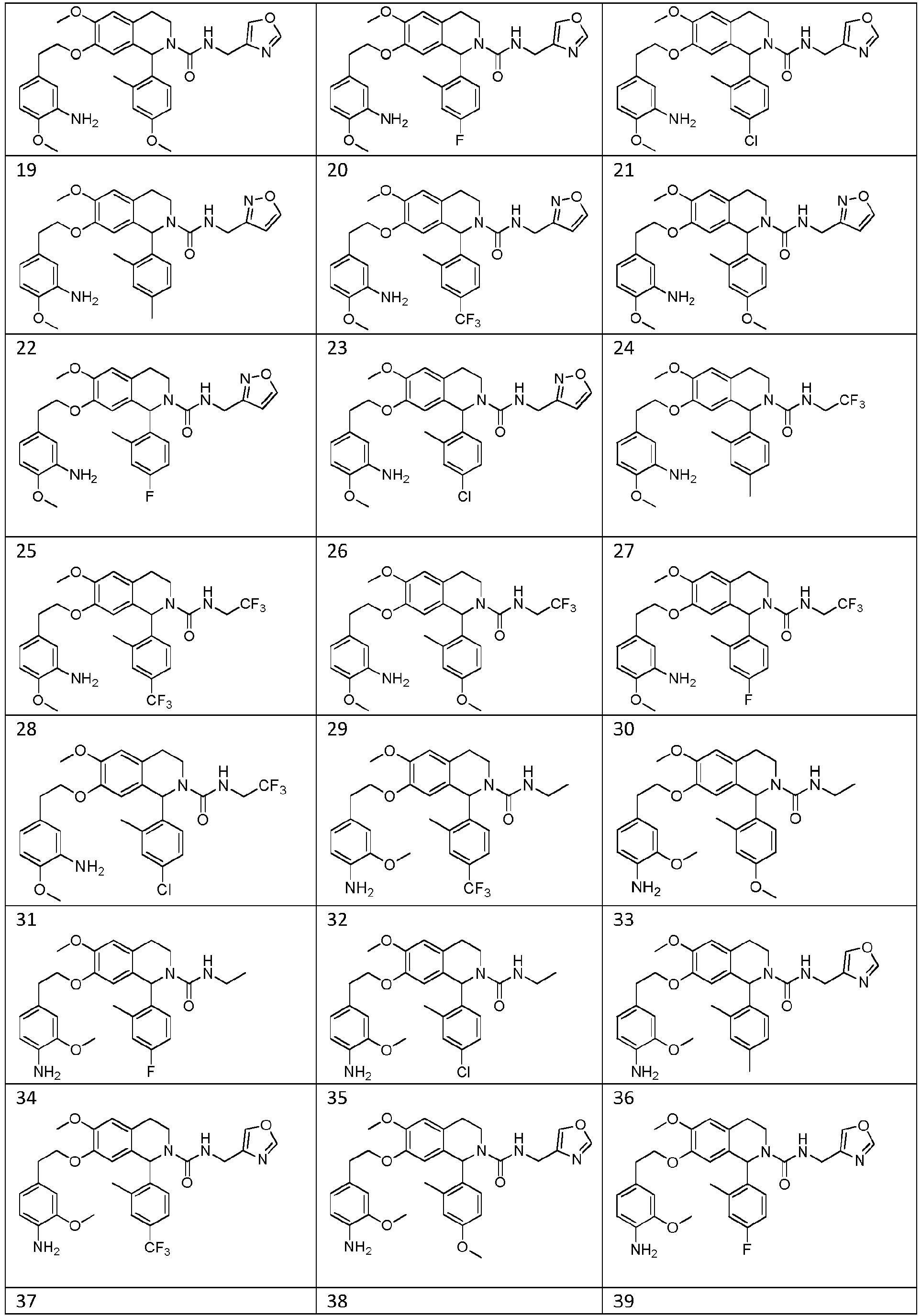

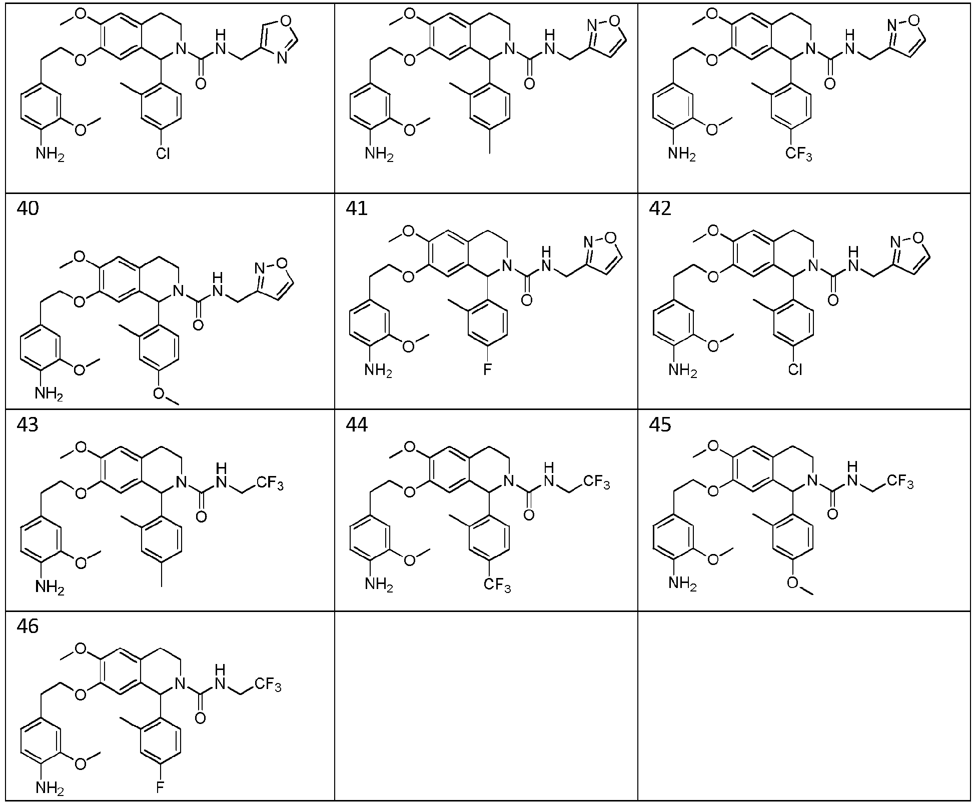

12. A pharmaceutical composition comprising at least one compound according to any one of claims 1 to 11 and a pharmaceutically acceptable carrier.

13. The compound according to any one of claims 1 to 11 or the composition according to claim 12 for use as a medicament.

14. The compound according to any one of claims 1 to 11 or the composition according to claim 12 for use in the treatment of cancer.

## Patentansprüche

1. Verbindung der Formel I, Enantiomere und pharmazeutisch akzeptable Salze derselben: 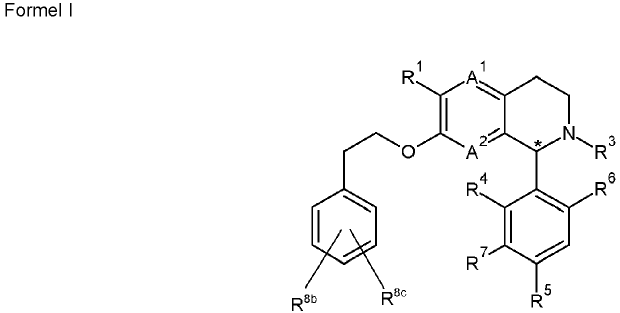 in welcher
A¹ aus CR² und N ausgewählt wird;
A² aus CH und N ausgewählt wird;
R¹ OR^{x} ist,
R^{x} C₁₋₄ Alkyl oder H ist;
R² H, C₁₋₄ Alkyl, C₁₋₄ Haloalkyl, Halogen, OC₁₋₄ Alkyl, OC₁₋₄ Haloalkyl, OH oder NH₂ ist;
R³-(CH₂)ₙ³-C(Y³)-(X³)ₘ³-(CH₂)ₖ³-R^{3a} ist,
n³ eine ganze Zahl im Bereich von 0 bis 2 ist,
Y³ O ist,
X³ NH oder O ist,
m³ 0 oder 1 ist,
k³ 0 oder 1 ist,
R^{3a} C₁₋₄ Alkyl, C₁₋₄ Haloalkyl oder Het³ ist,
Het³ Oxazolyl, Isoxazolyl, Thiazolyl, Isothiazolyl oder Pyridinyl ist;
R⁴ Halogen, C₁₋₄ Alkyl, C₁₋₄ Haloalkyl, OC₁₋₄ Alkyl oder OC₁₋₄ Haloalkyl ist;
R⁵ H, Halogen, OC₁₋₄ Alkyl, OH, C₁₋₄ Alkyl, C₁₋₄ Haloalkyl oder OC₁₋₄ Haloalkyl ist;
R⁶ H, OH oder Halogen ist;
R⁷ H, Halogen, OH oder C₁₋₄ Alkyl ist;
R^{8b} und R^{8c} unabhängig aus der Gruppe bestehend aus OH, C₁₋₄ Alkyl, NH₂, NO₂, OC₁₋₄ Alkyl und Halogen ausgewählt werden.

2. Verbindung nach einem der vorhergehenden Ansprüche, mit der Formel II, Enantiomere oder pharmazeutisch akzeptable Salze derselben: 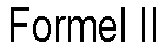 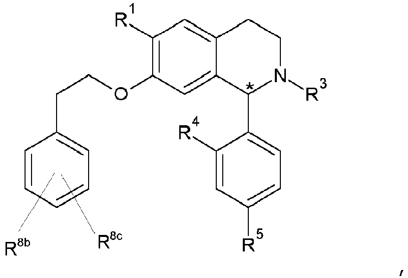 in welcher R¹, R³, R⁴, R⁵, R^{8b} und R^{8c} wie in einem vorhergehenden Anspruch definiert sind.

3. Verbindung nach einem der Ansprüche 1 oder 2, in welcher der Phenylring ein in Stellungen 3 und 4 substituiertes Phen-1-yl ist.

4. Verbindung nach einem der Ansprüche 1 bis 3, in welcher X³ NH ist.

5. Verbindung nach Anspruch 4, in welcher n³ 0 ist und m³ 1 ist.

6. Verbindung nach einem der Ansprüche 4 oder 5, in welcher R^{3a} Oxazolyl, Methyl oder Trifluormethyl ist.

7. Verbindung nach einem der Ansprüche 4 bis 6, in welcher k³ 1 ist.

8. Verbindung nach einem der Ansprüche 1 bis 7, in welcher A¹ CH ist.

9. Verbindung nach einem der Ansprüche 1 bis 8, in welcher R⁶ und R⁷ H sind.

10. Verbindung nach einem der Ansprüche 1 bis 9, in welcher R⁵ F, Cl, CF₃, CH₃ oder OCH₃ ist.

11. Verbindung nach einem der vorhergehenden Ansprüche, welche aus den Folgenden ausgewählt wird:

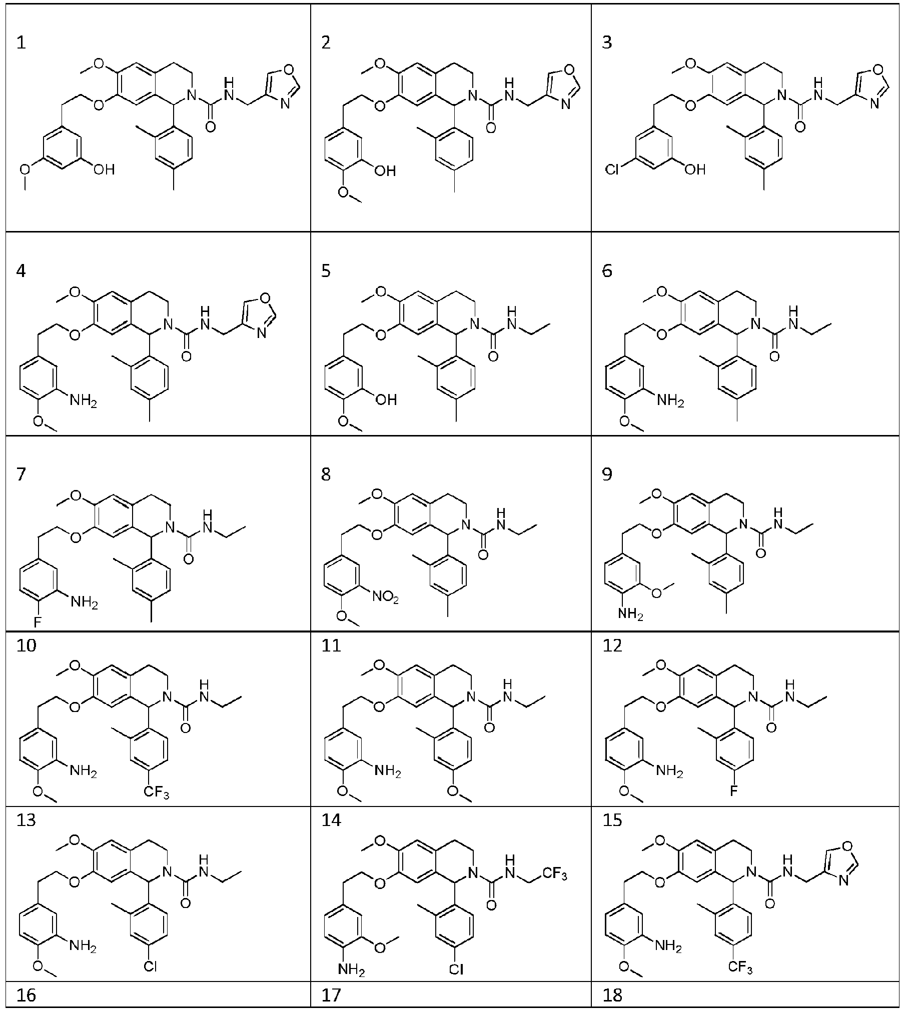

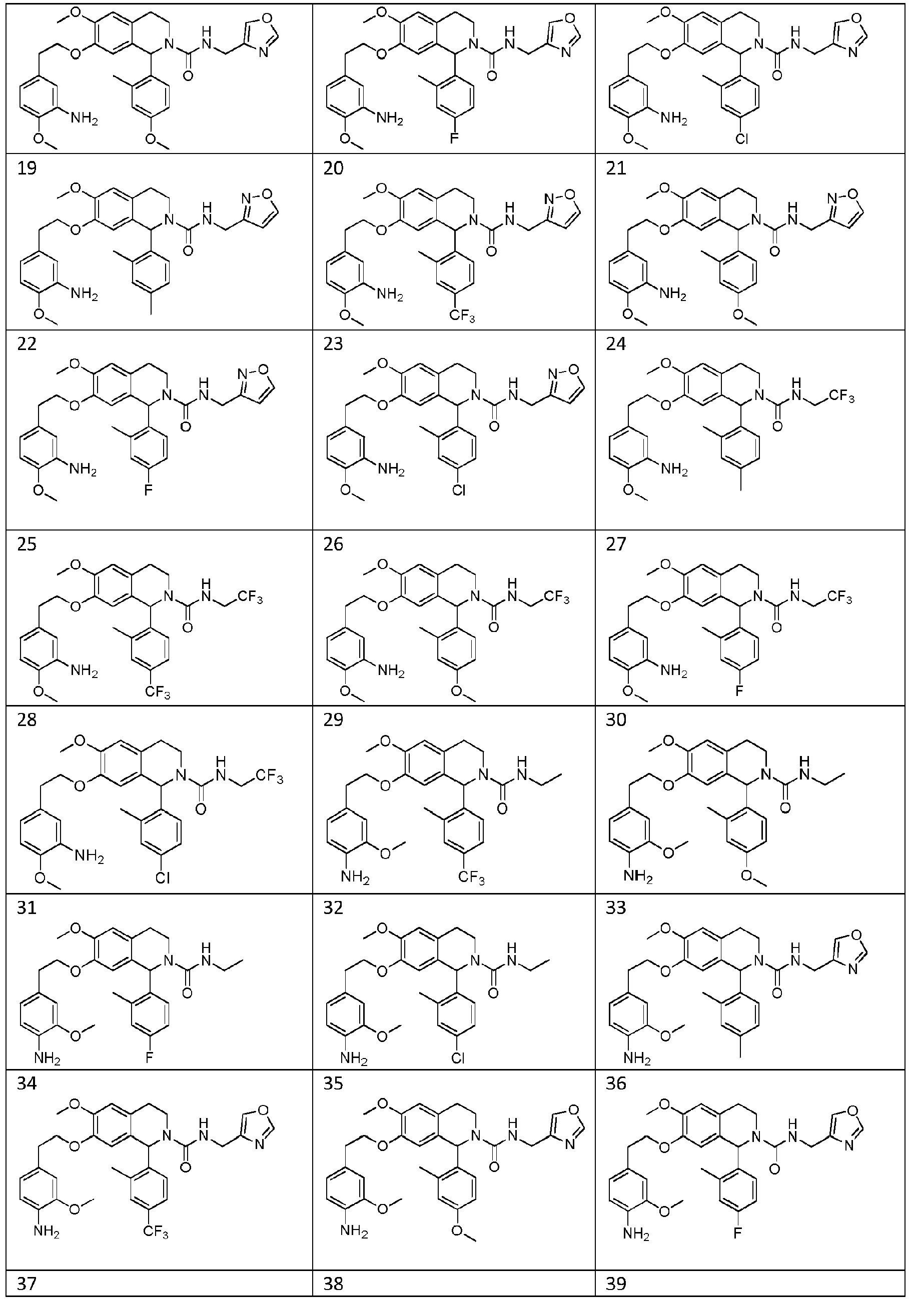

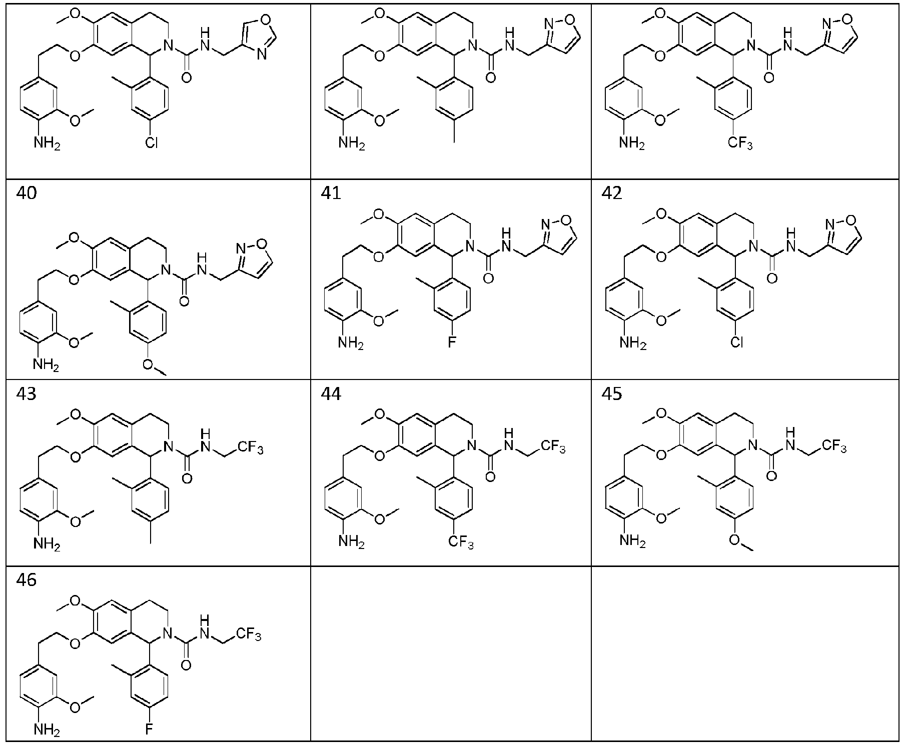

12. Pharmazeutische Zusammensetzung umfassend mindestens eine Verbindung nach einem der Ansprüche 1 bis 11 und einen pharmazeutisch akzeptablen Träger.

13. Verbindung nach einem der Ansprüche 1 bis 11 oder Zusammensetzung nach Anspruch 12 für deren Verwendung als Medikament.

14. Verbindung nach einem der Ansprüche 1 bis 11 oder Zusammensetzung nach Anspruch 12 für deren Verwendung bei der Behandlung von Krebs.

## Revendications

1. Composé de formule I, énantiomères et sels pharmaceutiquement acceptables de celui-ci : 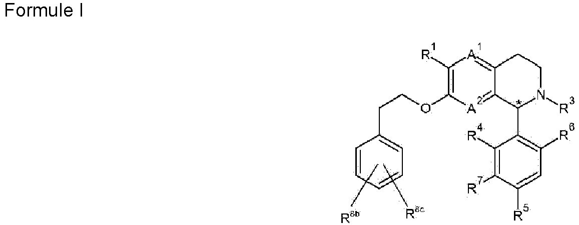 dans lequel
A¹ est sélectionné de CR² et N ;
A² est sélectionné de CH et N ;
R¹ est OR^{x},
R^{x} est alkyle C₁₋₄ ou H ;
R² est H, alkyle C₁₋₄, haloalkyle C₁₋₄, halogène, O-alkyle C₁₋₄, O_haloalkyle C₁₋₄, OH ou NH_{2 ;}
R³ est -(CH₂)ₙ³-C(Y³)-(X³)ₘ³-(CH₂)ₖ³-R^{3a},
n³ est un nombre entier dans la plage allant de 0 à 2,
Y³ est O,
X³ est NH ou O,
m³ est 0 ou 1,
k³ est 0 ou 1,
R^{3a} est alkyle C₁₋₄, haloalkyle C₁₋₄ ou Het³,
Het³ est oxazolyle, isoxazolyle, thiazolyle, isothiazolyle ou pyridinyle ;
R⁴ est halogène, alkyle C₁₋₄, haloalkyle C₁₋₄, O-alkyle C₁₋₄ ou O-haloalkyle C₁₋₄ ;
R⁵ est H, halogène, O-alkyle C₁₋₄, OH, alkyle C₁₋₄, haloalkyle C₁₋₄ ou O- haloalkyle C₁₋₄ ;
R⁶ est H, OH ou halogène ;
R⁷ est H, halogène, OH ou alkyle C₁₋₄ ;
R^{8b} et R^{8c} sont sélectionnés indépendamment du groupe constitué par OH, alkyl C₁₋₄, NH₂, NO₂, O-alkyle C₁₋₄ et halogène.

2. Composé selon l'une quelconque des revendications précédentes, ayant la formule II, énantiomères ou sels pharmaceutiquement acceptables de celui-ci : 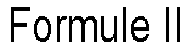 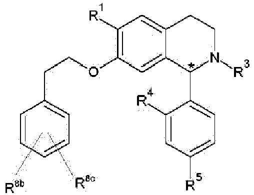 dans lequel R¹, R³, R⁴, R⁵, R^{8b} et R^{8c} sont tel que définis dans l'une quelconque des revendications précédentes.

3. Composé selon revendication 1 ou 2, dans lequel l'anneau phényle est un phen-1-yl substitué en positions 3 et 4.

4. Composé selon l'une quelconque des revendications 1 à 3, dans lequel X³ est NH.

5. Composé selon la revendication 4, dans lequel n³ est 0 et m³ est 1.

6. Composé selon l'une quelconque des revendications 4 ou 5, dans lequel R^{3a} est oxazolyle, méthyle ou trifluorométhyle.

7. Composé selon l'une quelconque des revendications 4 à 6, dans lequel k³ est 1.

8. Composé selon l'une quelconque des revendications 1 à 7, dans lequel A¹ est CH.

9. Composé selon l'une quelconque des revendications 1 à 8, dans lequel R⁶ et R⁷ sont H.

10. Composé selon l'une quelconque des revendications 1 à 9, dans lequel R⁵ est F, Cl, CF₃, CH₃ ou OCH₃.

11. Composé selon l'une quelconque des revendications précédentes, qui est sélectionnée de :

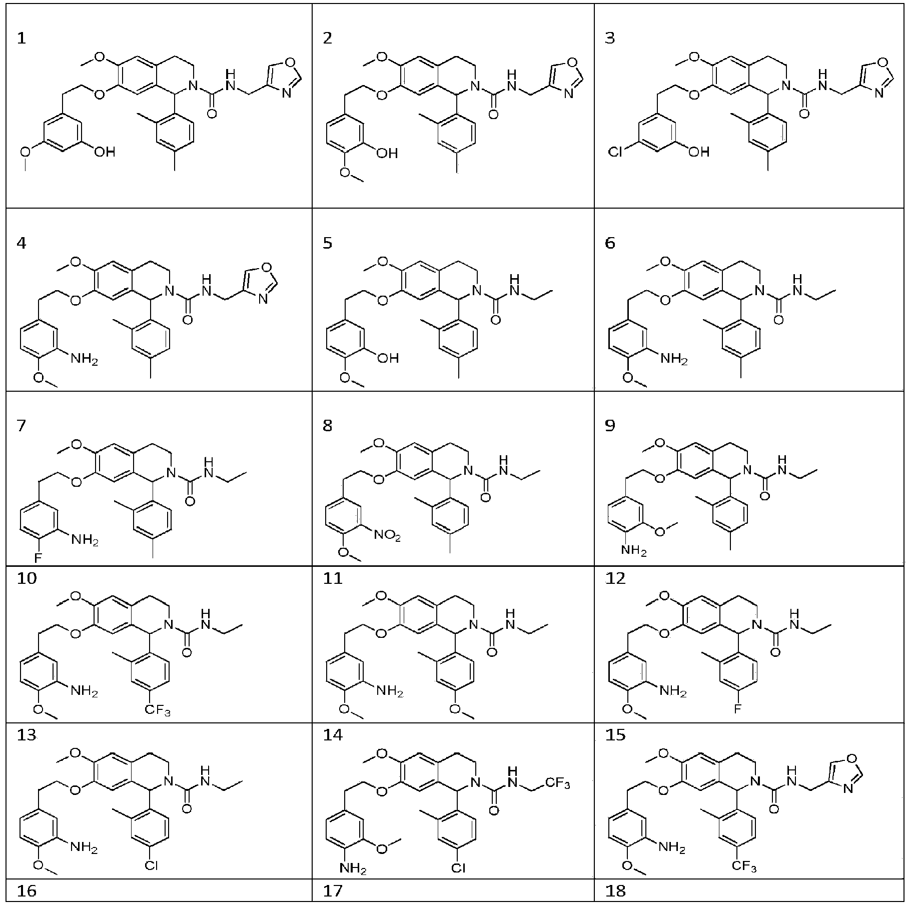

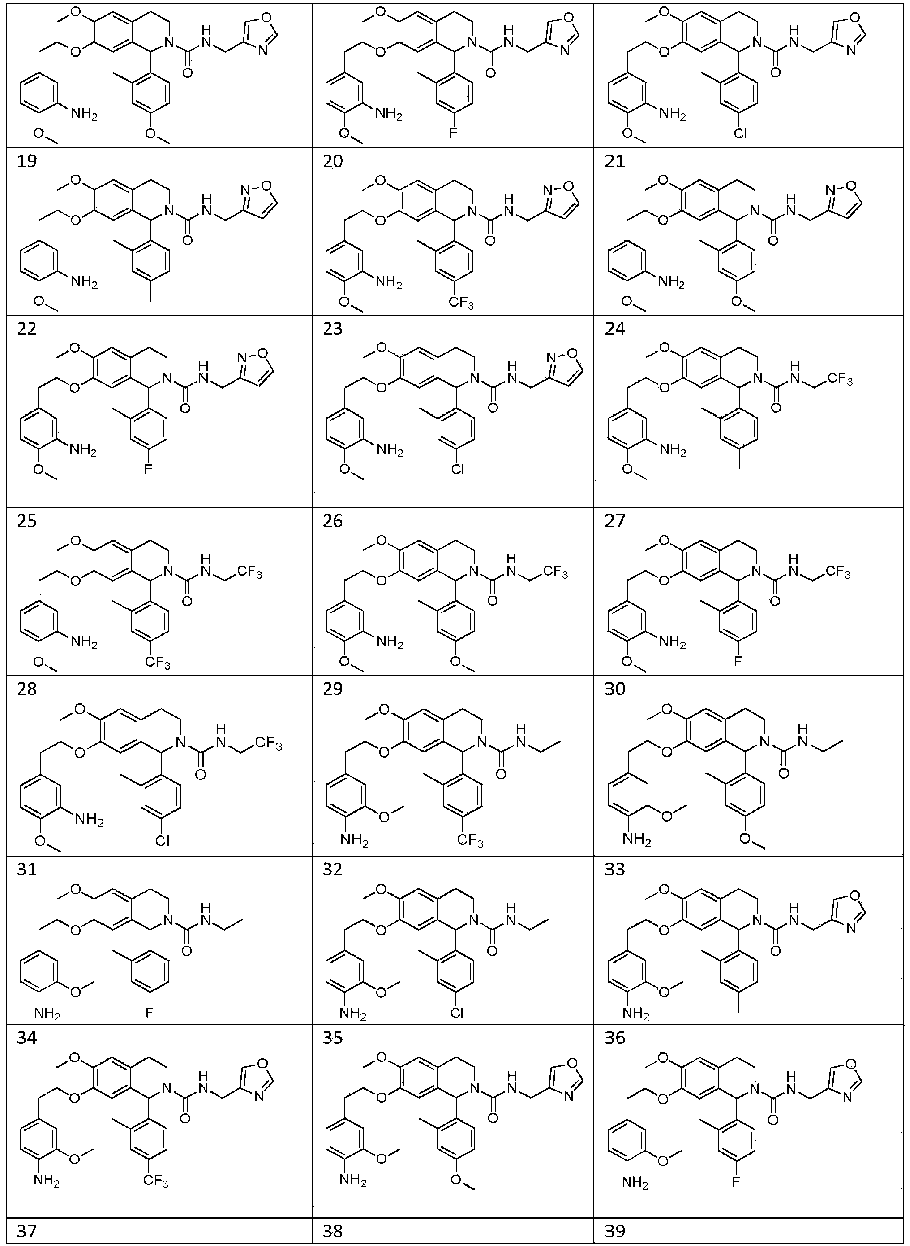

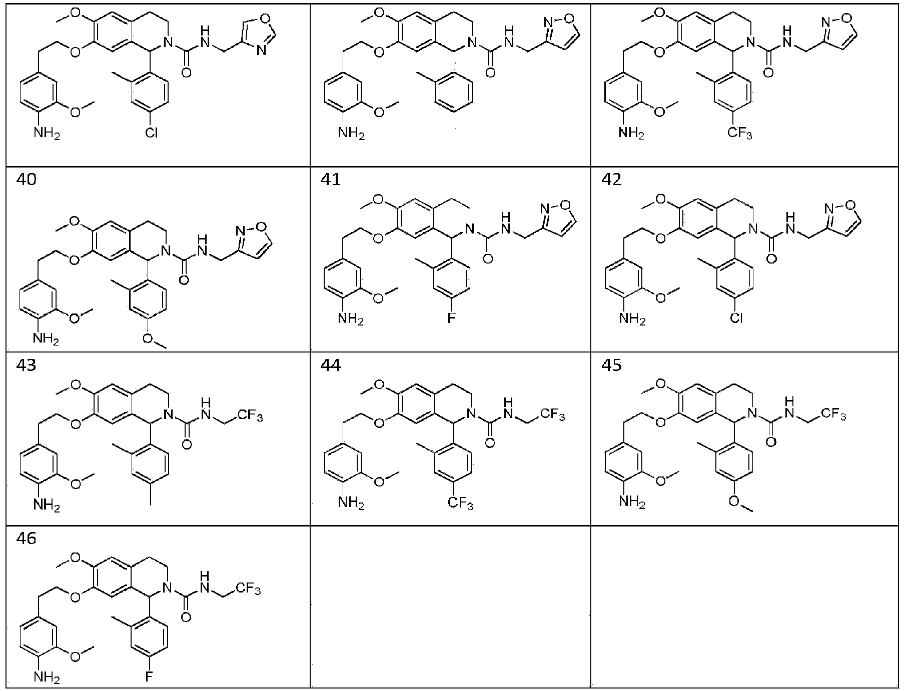

12. Composition pharmaceutique comprenant au moins un composé selon l'une quelconque des revendications 1 à 11 et un véhicule pharmaceutiquement acceptable.

13. Composé selon l'une quelconque des revendications 1 à 11 ou composition selon la revendication 12 pour son utilisation en tant que médicament.

14. Composé selon l'une quelconque des revendications 1 à 11 ou composition selon la revendication 12 pour son utilisation dans le traitement de cancer.
